(19) Europäisches Patentamt / European Patent Office / Office européen des brevets

(11) **EP 1 776 591 B1**

(12) **EUROPEAN PATENT SPECIFICATION**

(45) Date of publication and mention of the grant of the patent:
**16.10.2013 Bulletin 2013/42**

(21) Application number: **05777801.1**

(22) Date of filing: **08.08.2005**

(51) Int Cl.:
***G01N 33/68*** (2006.01) ***C12N 15/85*** (2006.01)
***A61K 49/00*** (2006.01) ***C12Q 1/68*** (2006.01)

(86) International application number:
**PCT/EP2005/053896**

(87) International publication number:
**WO 2006/015976 (16.02.2006 Gazette 2006/07)**

(54) **DIAGNOSTIC AND THERAPEUTIC USE OF A PLASMA MEMBRANE ATPASE**

DIAGNOSTISCHE UND THERAPEUTISCHE VERWENDUNG EINER PLASMAMEMBRAN-ATPASE

APPLICATIONS DIAGNOSTIQUES ET THERAPEUTIQUES D'UNE ATPASE DE LA MEMBRANE PLASMIQUE

(84) Designated Contracting States:
**AT BE BG CH CY CZ DE DK EE ES FI FR GB GR HU IE IS IT LI LT LU LV MC NL PL PT RO SE SI SK TR**

(30) Priority: **11.08.2004 US 600379 P**

(43) Date of publication of application:
**25.04.2007 Bulletin 2007/17**

(73) Proprietor: **Evotec International GmbH**
**22419 Hamburg (DE)**

(72) Inventors:
- **VON DER KAMMER, Heinz**
  **22607 Hamburg (DE)**
- **POHLNER, Johannes**
  **22175 Hamburg (DE)**

(74) Representative: **von Kreisler Selting Werner**
**Deichmannhaus am Dom**
**Bahnhofsvorplatz 1**
**50667 Köln (DE)**

(56) References cited:

- **OKUNADE GBOLAHAN W ET AL: "Targeted ablation of plasma membrane Ca2+-ATPase (PMCA) 1 and 4 indicates a major housekeeping function for PMCA1 and a critical role in hyperactivated sperm motility and male fertility for PMCA4" JOURNAL OF BIOLOGICAL CHEMISTRY, vol. 279, no. 32, 3 June 2004 (2004-06-03), pages 33742-33750, XP002363283 ISSN: 0021-9258**
- **HILFIKER HELENE ET AL: "Structure of the gene encoding the human plasma membrane calcium pump isoform 1" JOURNAL OF BIOLOGICAL CHEMISTRY, vol. 268, no. 26, 1993, pages 19717-19725, XP002363284 ISSN: 0021-9258 cited in the application**
- **POST STEPHEN G: "Future scenarios for the prevention and delay of Alzheimer disease onset in high-risk groups: An ethical perspective" AMERICAN JOURNAL OF PREVENTIVE MEDICINE, vol. 16, no. 2, February 1999 (1999-02), pages 105-110, XP002363285 ISSN: 0749-3797**
- **RONQUIST G ET AL: "Imbalance of plasma membrane ion leak and pump relationship as a new aetiological basis of certain disease states." JOURNAL OF INTERNAL MEDICINE, vol. 254, no. 6, December 2003 (2003-12), pages 517-526, XP002363286 ISSN: 0954-6820**
- **ZACHARIAS DAVID A ET AL: "MRNA expression of the four isoforms of the human plasma membrane Ca-2+-ATPase in the human hippocampus" MOLECULAR BRAIN RESEARCH, vol. 45, no. 1, 1997, pages 173-176, XP002363287 ISSN: 0169-328X**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 776 591 B1

## Description

The present invention relates to methods of diagnosing, prognosticating and monitoring the progression of neurodegenerative diseases in a subject. Furthermore, methods of therapy control and screening for modulating agents of neurodegenerative diseases are provided. The invention also discloses pharmaceutical compositions, the use of a kit, recombinant animal models and the use of said recombinant animal models.

Neurodegenerative diseases, in particular Alzheimer's disease (AD), have a strongly debilitating impact on a patient's life. Furthermore, these diseases constitute an enormous health, social, and economic burden. AD is the most common neurodegenerative disease, accounting for about 70 % of all dementia cases, and it is probably the most devastating age-related neurodegenerative condition affecting about 10 % of the population over 65 years of age and up to 45 % over age 85 (Vickers et al., Progress in Neurobiology 2000, 60: 139-165; Walsh and Selkoe, Neuron 2004, 44:181-193). Presently, this amounts to an estimated 12 million cases in the US, Europe, and Japan. This situation will inevitably worsen with the demographic increase in the number of old people in developed countries. The neuropathological hallmarks that occur in the brains of individuals with AD are senile plaques, composed of amyloid-β protein, and profound cytoskeletal changes coinciding with the appearance of abnormal filamentous structures and the formation of neurofibrillary tangles.

The amyloid-β protein evolves from the cleavage of the amyloid precursor protein (APP) by different kinds of proteases (Selkoe and Kopan, Annu Rev Neurosci 2003, 26:565-597; Ling et al., Int J Biochem Cell Biol 2003, 35: 1505-1535). Two types of plaques, diffuse plaques and neuritic plaques can be detected in the brain of AD patients. They are primarily found in the cerebral cortex and hippocampus. The generation of toxic Aβ deposits in the brain starts very early in the course of AD, and it is discussed to be a key player for the subsequent destructive processes leading to AD pathology. The other pathological hallmarks of AD are neurofibrillary tangles (NFTs) and abnormal neurites, described as neuropil threads (Braak and Braak, J Neural Transm 1998, 53: 127-140). NFTs emerge inside neurons and consist of chemically altered tau, which forms paired helical filaments twisted around each other. Along the formation of NFTs, a loss of neurons can be observed (Johnson and Jenkins, J Alzheimers Dis 1996, 1: 38-58; Johnson and Hartigan, J Alzheimers Dis 1999, 1: 329-351). The appearance of neurofibrillary tangles and their increasing number correlates well with the clinical severity of AD (Schmitt et al., Neurology 2000, 55: 370-376). AD is a progressive disease that is associated with early deficits in memory formation and ultimately leads to the complete erosion of higher cognitive function. The cognitive disturbances include among other things memory impairment, aphasia, agnosia and the loss of executive functioning. A characteristic feature of the pathogenesis of AD is the selective vulnerability of particular brain regions and subpopulations of nerve cells to the degenerative process. Specifically, the temporal lobe region and the hippocampus are affected early and more severely during the progression of the disease. On the other hand, neurons within the frontal cortex, occipital cortex, and the cerebellum remain largely intact and are protected from neurodegeneration (Terry et al., Annals of Neurology 1981, 10: 184-92).

Currently, there is no cure for AD, nor is there an effective treatment to halt the progression of AD or even to diagnose AD ante- mortem with high probability. Several risk factors have been identified that predispose an individual to develop AD, among them most prominently the epsilon 4 allele of the three different existing alleles (epsilon 2, 3, and 4) of the apolipoprotein E gene (ApoE) (Strittmatter et al., Proc Natl Acad Sci USA 1993, 90: 1977- 81; Roses, Ann NY Acad Sci 1998, 855: 738- 43) . Although there are rare examples of early- onset AD which have been attributed to genetic defects in the genes for amyloid precursor protein (APP) on chromosome 21, presenilin- 1 on chromosome 14, and presenilin- 2 on chromosome 1, the prevalent form of late- onset sporadic AD is of hitherto unknown etiologic origin. The late onset and complex pathogenesis of neurodegenerative disorders pose a formidable challenge to the development of therapeutic and diagnostic agents. It is crucial to expand the pool of potential drug targets and diagnostic markers. It is therefore an object of the present invention to provide insight into the pathogenesis of neurological diseases and to provide methods, materials, agents, compositions, and animal models which are suited inter alia for the diagnosis and development of a treatment of these diseases. This object has been solved by the features of the independent claims. The subclaims define preferred embodiments of the present invention.

The present invention is based on the detection of dysregulated, differential expression of a gene coding for calcium pumps of the plasma membrane (plasma membrane $Ca^{2+}$-ATPases or ATP2B or PMCAs) and the proteins thereof in human Alzheimer's disease brain samples.

PMCAs are responsible for the expulsion of $Ca^{2+}$ from the cytosol of all eukaryotic cells. Together with $Na^{+}$/$Ca^{2+}$ exchangers, they are the major plasma membrane transport system responsible for the long-term regulation of the resting intracellular $Ca^{2+}$ concentration. The PMCAs belong to the family of P-type primary ion transport ATPases characterized by the formation of an aspartyl phosphate intermediate during the reaction cycle. Mammalian PMCAs are encoded by four separate genes, and additional isoform variants are generated via alternative RNA splicing of the primary gene transcripts. The expression of different PMCA isoforms and splice variants is regulated in a developmental, tissue- and cell type-specific manner, suggesting that these pumps are functionally adapted to the physiological needs of particular cells and tissues. Alternative splicing affects two major locations in the plasma membrane $Ca^{2+}$ pump protein:

the first intracellular loop and the COOH-terminal tail. These two regions correspond to major regulatory domains of the pumps. In the first cytosolic loop, the affected region is embedded between a putative G protein binding sequence and the site of phospholipid sensitivity, and in the COOH-terminal tail, splicing affects pump regulation by calmodulin, phosphorylation, and differential interaction with PDZ domain-containing anchoring and signaling proteins. The identification of mice carrying PMCA mutations that lead to diseases such as hearing loss and ataxia, as well as the corresponding phenotypes of genetically engineered PMCA "knockout" mice further support the concept of specific, nonredundant roles for each $Ca^{2+}$ pump isoform in cellular $Ca^{2+}$ regulation (Strehler & Zacharias, Physiological Reviews 2001, 81: 21-50). The complete structure of the gene for the human plasma membrane calcium ATPase isoform 1 (ATP2B1 or PMCA1) has been elucidated in 1993 (Hilfiker et al., Journal Biological Chemistry 1993, 268: 19717-19725). The protein is encoded by 21 exons covering more than 100 kilobases (kb) of DNA. An intron over 35 kb separates the 5'untranslated exon 1 from the exon containing the translational start codon. The entire putative promoter and 5'flanking region is embedded in a CpG island and is characterized by the presence of numerous Sp1 factor-binding sequences and by the absence of a TATA box. In accordance with the ubiquitous tissue distribution of its mRNA these results suggest that the hPMCA1 gene is of the housekeeping type.

Stauffer et al. analysed alternative splicing options and the quantitative tissue distribution of the transcripts of the four currently known human plasma membrane calcium pump (PMCA) genes in seven tissues (cerebral cortex, skeletal and heart muscle, stomach, liver, lung, and kidney). The mRNAs of genes 1 and 4 were found to be present in similar amounts in all tissues, whereas the transcripts of genes 2 and 3 were expressed in a tissue-specific manner, i.e. their amounts were highest in fetal skeletal muscle and brain. Alternative splicing was found to occur in the PMCA transcripts at two major regulatory sites (sites A and C), adjacent to the amino-terminal phospholipid-responsive region and within the carboxyl-terminal calmodulin binding domain, respectively. Novel splicing variants not described previously for human genes were detected for hPMCA3 and 4 at site A and for hPMCA1, 2, and 3 at site C. For all genes a common splice variant was found at both splice sites. The common splice variant at site A was characterized by the inclusion of a small exon (hPMCA1, 39 base pairs (bp); hPMCA2, 42 bp; hPMCA3, 42 bp; hPMCA4, 36 bp). In the common splice variant at site C, an exon (hPMCA1, 154 bp; hPMCA2, 227 bp; hPMCA3, 164 bp; hPMCA4, 178 bp) was excluded in the mRNA. All genes normally express these main splice variants in all tissues in which the corresponding isoform is present. The splicing complexity at site C was found to be augmented in the transcripts of PMCA2 and PMCA3 through the use of additional exons, and in PMCA1 and 3 through the use of additional internal splice sites in the single alternatively spliced 164-base pair exon. (Stauffer et al., Journal Biological Chemistry 1993, 268: 25993-26003).

Among the tissues tested for expression of the PMCA isoforms by Stauffer at al., the cerebral cortex was found to display the highest complexity of transcripts, i.e. the cortex contained products from all four genes and virtually every alternative splicing option. mRNA of gene 2 was detected in considerable amounts only in the cerebral cortex (20% of all mRNAs), whereas that of gene 3 was present almost exclusively in fetal skeletal muscle and in cerebral cortex (6% of all transcripts) (Stauffer et al., JBC 1993, 268: 25993-26003). In general the cererbral cortex was found to contain very high relative amounts of the transcripts of each of these genes. PMCA1 seems to be the more abundant isoform, with an average ratio of 1:0.67 over PMCA 4 (Stauffer et al., JBC 1993, 268: 25993-26003).

Plasma membrane Ca2+ ATPase (PMCA) pump isoforms 2, 3, and 1a are expressed in large amounts in the cerebellum of adult rats but only minimally in neonatal cerebellum. 25 mM KCl-activated L-type Ca2+ channels, significantly increasing cytosolic Ca2+. Changes in the concentration of Ca2+ in the culturing medium affected the expression of the pumps.

Apart from the function of reducing intracellular calcium levels to below an activation threshold, PMCA1 has been shown to function in neurite extension in rat pheochromocytoma cells (Brandt et al., PNAS 1996, 93: 13843-13848). Blocking the production of PMCA1 in PC6 cells by using antisense RNA impairs their ability to extend neurites in response to nerve growth factor (NGF) directed neuronal cell differentiation. The inability to extend neurites was shown to be a consequence of down-regulating a 1-integrin expression in cells lacking plasma membrane $Ca^{2+}$-ATPase.

By overexpression of the four basic isoforms of the plasma membrane $Ca^{2+}$ pump and the two C- terminally truncated spliced variants PMCA4CII (4a) and 3CII (3a) in Chinese hamster ovary cells, Brini and coworkers demonstrated that the neuron specific isoforms PMCA2 and 3 are more effective in controlling homeostasis of $Ca^{2+}$ than the ubiquitous isoforms PMCA1 and 4 (Brini et al., Journal Biological Chemistry 2001, 278: 24500- 24508) . All four basic pump variants influenced the homeostasis of $Ca^{2+}$ in the native intracellular environment. The level of $[Ca^{2+}]$ in the endoplasmic reticulum and the height of the $[Ca^{2+}]$ transients generated in the cytosol and in the mitochondria by the emptying of the endoplasmic reticulum store by inositol 1, 4, 5- trisphosphate were all reduced by the overexpression of the pumps. The effects were much greater with the neuron- specific PMCA2 and PMCA3 than with the ubiquitously expressed isoforms 1 and 4. Unexpectedly, the truncated PMCA3 and PMCA4 were as effective as the full- length variants in influencing the homeostasis of $Ca^{2+}$ in the cytosol and the organelles. In particular, PMCA4CII (4a) was as effective as PMCA4CI (4b), even if its affinity for calmodulin is much lower. The results indicate that the availability of calmodulin may not be critical for the modulation of PMCA pumps *in vivo* (Brini et al., Journal Biological Chemistry 2001, 278: 24500- 24508) .

The singular forms "a", "an", and "the" as used herein and in the claims include plural reference unless the context dictates otherwise. For example, "a cell" means as well a plurality of cells, and so forth. The term "and/or" as used in the present specification and in the claims implies that the phrases before and after this term are to be considered either as alternatives or in combination. For instance, the wording "determination of a level and/or an activity" means that either only a level, or only an activity, or both a level and an activity are determined. The term "level" as used herein is meant to comprise a gage of, or a measure of the amount of, or a concentration of a transcription product, for instance an mRNA, or a translation product, for instance a protein or polypeptide. The term "activity" as used herein shall be understood as a measure for the ability of a transcription product or a translation product to produce a biological effect or a measure for a level of biologically active molecules. The term "activity" also refers to enzymatic activity or to biological activity and/or pharmacological activity which refers to binding, antagonization, repression, blocking, neutralization or sequestration of an ion channel or ion channel subunit and which refers to activation, agonization, upregulation of an ion channel or ion channel subunit. "Biological activity" includes but is not limited to the transmembrane transport of ions and/or transmembrane ion flow and/or the regulation thereof. "Pharmacological activity" includes but is not limited to the ability of an ion channel or an ion channel subunit to bind a ligand, a compound, an agent, a modulator and/or another ion channel subunit. The terms "level" and/or "activity" as used herein further refer to gene expression levels or gene activity. Gene expression can be defined as the utilization of the information contained in a gene by transcription and translation leading to the production of a gene product. "Dysregulation" shall mean an upregulation or downregulation of gene expression. A gene product comprises either RNA or protein and is the result of expression of a gene. The amount of a gene product can be used to measure how active a gene is. The term "gene" as used in the present specification and in the claims comprises both coding regions (exons) as well as non- coding regions (e.g. non- coding regulatory elements such as promoters or enhancers, introns, leader and trailer sequences) . The term "ORF" is an acronym for "open reading frame" and refers to a nucleic acid sequence that does not possess a stop codon in at least one reading frame and therefore can potentially be translated into a sequence of amino acids. "Regulatory elements" shall comprise inducible and non- inducible promoters, enhancers, operators, and other elements that drive and regulate gene expression. The term "fragment" as used herein is meant to comprise e.g. an alternatively spliced, or truncated, or otherwise cleaved transcription product or translation product. The term "derivative" as used herein refers to a mutant, or an RNA- edited, or a chemically modified, or otherwise altered transcription product, or to a mutant, or chemically modified, or otherwise altered translation product. For the purpose of clarity, a derivative transcript, for instance, refers to a transcript having alterations in the nucleic acid sequence such as single or multiple nucleotide deletions, insertions, or exchanges. A derivative translation product, for instance, may be generated by processes such as altered phosphorylation, or glycosylation, or acetylation, or lipidation, or by altered signal peptide cleavage or other types of maturation cleavage. These processes may occur post- translationally. The term "modulator" as used in the present invention and in the claims refers to a molecule capable of changing or altering the level and/or the activity of a gene, or a transcription product of a gene, or a translation product of a gene. Preferably, a "modulator" is capable of changing or altering the biological activity of a transcription product or a translation product of a gene. Said modulation, for instance, may be an increase or a decrease in the biological activity and/or pharmacological activity, in enzyme activity, a change in binding characteristics, or any other change or alteration in the biological, functional, or immunological properties of said translation product of a gene. A "modulator" refers to a molecule which has the capacity to either enhance or inhibit, thus to "modulate" a functional property of an ion channel subunit or an ion channel, to "modulate" binding, antagonization, repression, blocking, neutralization or sequestration of an ion channel or ion channel subunit and to "modulate" activation, agonization and upregulation. "Modulation" will be also used to refer to the capacity to affect the biological activity of a cell. The terms "agent", "reagent", or "compound" refer to any substance, chemical, composition, or extract that have a positive or negative biological effect on a cell, tissue, body fluid, or within the context of any biological system, or any assay system examined. They can be agonists, antagonists, partial agonists or inverse agonists of a target. Such agents, reagents, or compounds may be nucleic acids, natural or synthetic peptides or protein complexes, or fusion proteins. They may also be antibodies, organic or anorganic molecules or compositions, small molecules, drugs and any combinations of any of said agents above. They may be used for testing, for diagnostic or for therapeutic purposes. The terms "oligonucleotide primer" or "primer" refer to short nucleic acid sequences which can anneal to a given target polynucleotide by hybridization of the complementary base pairs and can be extended by a polymerase. They may be chosen to be specific to a particular sequence or they may be randomly selected, e.g. they will prime all possible sequences in a mix. The length of primers used herein may vary from 10 nucleotides to 80 nucleotides. "Probes" are short nucleic acid sequences of the nucleic acid sequences described and disclosed herein or sequences complementary therewith. They may comprise full length sequences, or fragments, derivatives, isoforms, or variants of a given sequence. The identification of hybridization complexes between a "probe" and an assayed sample allows the detection of the presence of other similar sequences within that sample. As used herein, "homolog or homology" is a term used in the art to describe the relatedness of a nucleotide or peptide sequence to another nucleotide or peptide sequence, which is determined by the degree of identity and/or similarity between said sequences compared. In the art, the terms "identity" and "similarity" mean the degree of polypeptide or polynucleotide sequence relatedness which are determined by matching a query sequence and other sequences of preferably the same type (nucleic acid or protein sequence) with each other. Preferred computer program methods to calculate and determine "identity" and "similarity" include, but are not limited to GCG BLAST (Basic Local Alignment Search Tool) (Altschul et al., J. Mol. Biol. 1990, 215: 403- 410; Altschul et al., Nucleic Acids Res. 1997, 25: 3389- 3402; Devereux et al., Nucleic Acids Res. 1984, 12: 387), BLASTN 2.0 (Gish W., http: // blast.wustl.edu, 1996- 2002), FASTA (Pearson and Lipman, Proc. Natl. Acad. Sci. USA 1988, 85: 2444- 2448), and GCG GelMerge which determines and aligns a pair of contigs with the longest overlap (Wilbur and Lipman, SIAM J. Appl. Math. 1984, 44: 557- 567; Needleman and Wunsch, J. Mol. Biol. 1970, 48: 443- 453) . The term "variant" as used herein refers to any polypeptide or protein, in reference to polypeptides and proteins disclosed in the present invention, in which one or more amino acids are added and/or substituted and/or deleted and/or inserted at the N- terminus, and/or the C- terminus, and/or within the native amino acid sequences of the native polypeptides or proteins of the present invention, but retains its essential properties. Furthermore, the term "variant" shall include any shorter or longer version of a polypeptide or protein. "Variants" shall also comprise a sequence that has at least about 80% sequence identity, more preferably at least about 90% sequence identity, and most preferably at least about 95% sequence identity with the amino acid sequences of an ATP2B protein, in particular ATP2B1, SEQ ID NO: 1. "Variants" include, for example, proteins with conservative amino acid substitutions in highly conservative regions. "Proteins and polypeptides" of the present invention include variants, isoforms, fragments and chemical derivatives of the protein comprising the amino acid sequences of an ATP2B protein, SEQ ID NO: 1, ATP2B1. Sequence variations shall be included wherein a codon are replaced with another codon due to alternative base sequences, but the amino acid sequence translated by the DNA sequence remains unchanged. This known in the art phenomenon is called redundancy of the set of codons which translate specific amino acids. Included shall be such exchange of amino acids which would have no effect on functionality, such as arginine for lysine, valine for leucine, asparagine for glutamine. Proteins and polypeptides can be included which can be isolated from nature or be produced by recombinant and/or synthetic means. Native proteins or polypeptides refer to naturally- occurring truncated or secreted forms, naturally occurring variant forms (e.g. splice- variants) and naturally occurring allelic variants. The term "isolated" as used herein is considered to refer to molecules or substances which have been changed and/or that are removed from their natural environment, i.e. isolated from a cell or from a living organism in which they normally occur, and that are separated or essentially purified from the coexisting components with which they are found to be associated in nature. This notion further means that the sequences encoding such molecules can be linked by the hand of man to polynucleotides to which they are not linked in their natural state and such molecules can be produced by recombinant and/or synthetic means. Even if for said purposes those sequences may be introduced into living or non- living organisms by methods known to those skilled in the art, and even if those sequences are still present in said organisms, they are still considered to be isolated. In the present invention, the terms "risk", "susceptibility", and "predisposition" are tantamount and are used with respect to the probability of developing a neurodegenerative disease, preferably Alzheimer's disease.

The term "AD" shall mean Alzheimer's disease. "AD- type neuropathology", "AD pathology" as used herein refers to neuropathological, neurophysiological, histopathological and clinical hallmarks, signs and symptoms as described in the instant invention and as commonly known from state- of- the- art literature (see: Iqbal, Swaab, Winblad and Wisniewski, Alzheimer's Disease and Related Disorders (Etiology, Pathogenesis and Therapeutics), Wiley & Sons, New York, Weinheim, Toronto, 1999; Scinto and Daffner, Early Diagnosis of Alzheimer's Disease, Humana Press, Totowa, New Jersey, 2000; Mayeux and Christen, Epidemiology of Alzheimer's Disease: From Gene to Prevention, Springer Press, Berlin, Heidelberg, New York, 1999; Younkin, Tanzi and Christen, Presenilins and Alzheimer's Disease, Springer Press, Berlin, Heidelberg, New York, 1998) . The term "Braak stage" or "Braak staging" refers to the classification of brains according to the criteria proposed by Braak and Braak (Braak and Braak, Acta Neuropathology 1991, 82: 239-259; Braak and Braak, J Neural Transm 1998, 53: 127- 140) . On the basis of the distribution of neurofibrillary tangles and neuropil threads, the neuropathologic progression of AD is divided into six stages (stage 0 to 6) . In the instant invention low Braak stages (0- 3) may represent persons which are not considered to suffer from Alzheimer's disease signs and symptoms ("controls"), and Braak stages 4 to 6 may represent persons already suffering from Alzheimer's disease ("AD patients") . The values obtained from "controls" are the "reference values" representing a "known health status" and the values obtained from "AD patients" are the "reference values" representing a "known disease status". The higher the Braak stage the more likely is the possibility to display signs and symptoms of AD or the risk to develop signs and symptoms of AD. For a neuropathological assessment, i.e. an estimation of the probability that pathological changes of AD are the underlying cause of dementia, a recommendation is given by Braak H. (www. alzforum.org) . Neurodegenerative diseases or disorders according to the present invention comprise Alzheimer's disease, Parkinson's disease, Huntington's disease, amyotrophic lateral sclerosis, Pick's disease, fronto- temporal dementia, progressive nuclear palsy, corticobasal degeneration, cerebro- vascular dementia, multiple system atrophy, argyrophilic grain dementia and other tauopathies, and mild- cognitive impairment. Further conditions involving neurodegenerative processes are, for instance, ischemic stroke, age- related macular degeneration, narcolepsy, motor neuron diseases, prion diseases, traumatic nerve injury and repair, and multiple sclerosis.

The present invention discloses the identification, the differential expression, a dysregulation of human plasma membrane calcium ATPase (ATP2B or PMCA) gene expression, preferably of the human plasma membrane calcium ATPase isoform 1 (ATP2B1 or PMCA1), in brain regions of Alzheimer's disease patients in comparison with each other and/or in comparison to age-matched control tissue samples of frontal cortex, of temporal cortex and of the hippocampus, respectively. No such dysregulation is observed in samples derived from age-matched, healthy controls. The present invention discloses that the gene expression for ATP2B1 is varied, is dysregulated in AD-affected brains, in that ATP2B1 mRNA levels are decreased, are down-regulated in the temporal cortex as compared to the frontal cortex, or are elevated, are up-regulated in the frontal cortex as compared to the temporal cortex or the hippocampus. Further, the present invention discloses that the ATP2B1 expression differs between the frontal cortex and the temporal cortex of healthy age-matched control subjects compared to the frontal cortex and the temporal cortex of AD patients in that the level of ATP2B1 is downregulated in the temporal and frontal cortex of AD patients. No such dysregulation is observed comparing samples with each other obtained from age-matched, healthy controls. This dysregulation presumably relates to a pathologic alteration of ATP2B1 in AD-affected brains.

To date, no experiments have been described that demonstrate a relationship between the dysregulation of ATP2B1 gene expression and the pathology of neurodegenerative diseases, in particular AD. Likewise, no mutations in the ATP2B1 gene or protein have been described to be associated with said diseases. Linking the ATP2B1 gene and protein to such diseases, as disclosed in the instant invention, offers new ways, inter alia, for the diagnosis and treatment of said diseases.

The present invention discloses a dysregulation of a gene coding for ATP2B1 in specific brain regions of AD patients. Neurons within the inferior temporal lobe, the entorhinal cortex, the hippocampus, and the amygdala are subject to degenerative processes in AD (Terry et al., Annals of Neurology 1981, 10:184-192). These brain regions are mostly involved in the processing of learning and memory functions and display a selective vulnerability to neuronal loss and degeneration in AD. In contrast, neurons within the frontal cortex, the occipital cortex, and the cerebellum remain largely intact and preserved from neurodegenerative processes. Brain tissues from the frontal cortex (F), the temporal cortex (T) and the hippocampus (H) of AD patients and healthy, age-matched control individuals were used for the herein disclosed examples. Consequently, the ATP2B1 gene and its corresponding transcription and/or translation products have a causative role in the regional selective neuronal degeneration typically observed in AD. Alternatively, ATP2B1 may confer a neuroprotective function to the remaining surviving nerve cells. Based on these disclosures, the present invention has utility for the diagnostic evaluation and prognosis as well as for the identification of a predisposition to a neurodegenerative disease, in particular AD. Furthermore, the present invention provides methods for the diagnostic monitoring of patients undergoing treatment for such a disease.

In one aspect, the invention features a method of diagnosing or prognosticating Alzheimer's in a subject, or determining whether a subject is at increased risk of developing said disease or of monitoring the effect of a treatment administered to a subject having said disease. The method comprises: determining a level or an activity, or both said level and said activity of (i) a transcription product of the gene coding for the plasma membrane calcium ATPase (ATP2B1) having SEQ ID NO:1, and/or of (ii) a translation product of the gene coding for the plasma membrane calcium ATPase (ATP2B1) having SEQ ID NO:1 , in a sample obtained from said subject and comparing said level and/or said activity to a reference value representing a known disease status (patient), and/or to a reference value representing a known health status (control), and said level and/or said activity is varied compared to a reference value representing a known health status, and/or is similar or equal to a reference value representing a known disease status , thereby diagnosing or prognosticating said Alzheimer's disease in said subject, or determining whether said subject is at increased risk of developing said disease. The wording "in a subject" refers to results of the methods disclosed as far as they relate to a disease afflicting a subject, that is to say, said disease being "in" a subject.

The invention also discloses the construction and the use of primers and probes which are unique to the nucleic acid sequences, or fragments, or variants thereof, as disclosed in the present invention. The oligonucleotide primers and/or probes can be labeled specifically with fluorescent, bioluminescent, magnetic, or radioactive substances. The invention further relates to the detection and the production of said nucleic acid sequences, or fragments and variants thereof, using said specific oligonucleotide primers in appropriate combinations. PCR-analysis, a method well known to those skilled in the art, can be performed with said primer combinations to amplify said gene specific nucleic acid sequences from a sample containing nucleic acids. Such sample may be derived either from healthy or diseased subjects or subjects with defined braak stages. Whether an amplification results in a specific nucleic acid product or not, and whether a fragment of different length can be obtained or not, may be indicative for a neurodegenerative disease, in particular Alzheimer's disease. Thus, the invention provides nucleic acid sequences, oligonucleotide primers, and probes of at least 10 bases in length up to the entire coding and gene sequences, useful for the detection of gene mutations and single nucleotide polymorphisms in a given sample comprising nucleic acid sequences to be examined, which may be associated with neurodegenerative diseases, in particular Alzheimer's disease. This feature has utility for developing rapid DNA-based diagnostic tests, preferably also in the format of a kit.

Disclosed is a method of monitoring the progression of a neurodegenerative disease in a subject. A level, expression or an activity, or both said level, expression and said activity, of (i) a transcription product of the gene coding for a plasma membrane calcium ATPase (ATP2B1), and/or of (ii) a translation product of the gene coding for a plasma membrane calcium ATPase (ATP2B1), and/or of (iii) a fragment, or derivative, or variant of said transcription or translation product in a sample obtained from said subject is determined. Said level, expression and/or said activity is compared to a reference value representing a known disease or health status or a known Braak stage. Thereby, the progression of said neurodegenerative disease in said subject is monitored.

Further disclosed is a method of evaluating a treatment for a neurodegenerative disease, comprising determining a level, expression or an activity, or both said level, expression and said activity of (i) a transcription product of the gene coding for a plasma membrane calcium ATPase (ATP2B1), and/or of (ii) a translation product of the gene coding for a plasma membrane calcium ATPase (ATP2B1), and/or of (iii) a fragment, or derivative, or variant of said transcription or translation product in a sample obtained from a subject being treated for said disease. Said level, expression or said activity, or both said level, expression and said activity are compared to a reference value representing a known disease or health status or a known Braak stage, thereby evaluating the treatment for said neurodegenerative disease.

In a preferred embodiment of the herein claimed methods, kits, recombinant animals, molecules, assays, and uses of the instant invention, said ATP2B gene coding for a calcium pump of the plasma membrane is the gene coding for a plasma membrane calcium ATPase (ATP2B or PMCA), the ATP2B proteins. In a further preferred embodiment, said ATP2B gene is coding for a plasma membrane calcium ATPase isoform 1 or 2 or 3 or 4, also named variant 1 or 2 or 3 or 4. It is preferred that said ATP2B gene is coding for the plasma membrane calcium ATPase isoform 1 (ATP2B1 or PMCA1) or variant 1. The ATP2B isoform 1 is represented by the ATP2B1 gene (Genbank accession number L14561) coding for the protein of SEQ ID NO: 1, ATP2B1. The amino acid sequence of said protein is deduced from the mRNA sequence corresponding to SEQ ID NO: 2, ATP2B1 cDNA which corresponds to the cDNA consensus sequence constructed from Genbank accession numbers J04027, M95541, M95542, AK024895, AK027053, S49852 and several ESTs (see Figure 10). In the instant invention ATP2B1 also refers to the nucleic acid sequences of SEQ ID NO: 2, coding for the protein of SEQ ID NO: 1. In the instant invention ATP2B1 also refers to the nucleic acid sequence SEQ ID NO: 4 representing the coding sequence (cds) of human ATP2Bl. In the instant invention said sequences are "isolated" as the term is employed herein. Further, in the instant invention, the gene coding for said ATP2B or ATP2Bl protein is also generally referred to as the ATP2B1 gene or simply ATP2B1. Furtherance, the protein of ATP2B or ATP2B1 is also generally referred to as the ATP2B1 protein or simply ATP2B1.

In a further preferred embodiment of the herein claimed methods, kits, recombinant animals, molecules, assays, and uses of the instant invention, said neurodegenerative disease or disorder is Alzheimer's disease (AD), and said subjects suffer from signs and symptoms of Alzheimer's disease.

It is preferred that the sample to be analyzed and determined is selected from the group comprising brain tissue or other tissues, or body cells. The sample can also comprise cerebrospinal fluid or other body fluids including saliva, urine, stool, blood, serum plasma, or mucus. Preferably, the methods of diagnosis, prognosis, monitoring the progression or evaluating a treatment for Alzheimeir's disease, according to the instant invention, can be practiced *ex corpore,* they are practiced "in vitro" and such methods preferably relate to samples, for instance, body fluids or cells, removed, collected, or isolated from a subject or patient.

In further preferred embodiments, said reference value is that of a level, of expression or of an activity, or both said level and said activity of (i) a transcription product of the gene coding for an ATP2B1 protein, and/or of (ii) a translation product of the gene coding for an ATP2B1 protein, in a sample obtained from a subject not suffering from said disease (control sample, control) or in a sample obtained from a subject suffering from Alzheimer's disease (patient sample, patient), or from a person with a defined Braak stage.

In preferred embodiments, an alteration in the level, expression and/or activity, a varied level, expression and/or activity of a transcription product of the gene coding for ATP2B1 protein and/or of a translation product of the gene coding for ATP2B1 protein in a sample cell, or tissue, or body fluid obtained from said subject (patient sample) relative to a reference value representing a known health status (control sample) indicates a diagnosis, or prognosis, or increased risk of becoming diseased with AD.

In further preferred embodiments, an equal or similar level, expression and/or activity of a transcription product of the gene coding for ATP2B1 protein and/or of a translation product of the gene coding for ATP2B1 protein in a sample cell, or tissue, or body fluid obtained from said subject (patient sample) relative to a reference value representing a known disease status of ALzheimer's disease, indicates a diagnosis, or prognosis, or increased risk of becoming diseased with said neurodegenerative disease.

In another further preferred embodiment, an equal or similar level and/or activity of a transcription product of the gene coding for a ATP2B1 protein and/or of a translation product of the gene coding for a ATP2B1 protein in a sample cell, or tissue, or body fluid obtained from a subject relative to a reference value representing a known Braak stage which Braak stage reflects a high risk of developing signs and symptoms of AD, indicates a diagnosis, or prognosis, or an increased risk of becoming diseased with AD.

In preferred embodiments, measurement of the level of transcription products of the gene coding for an ATP2B1 protein is performed in a sample obtained from a subject using a quantitative PCR-analysis with primer combinations to amplify said gene specific sequences from cDNA obtained by reverse transcription of RNA extracted from a sample of a subject. Primer combinations (SEQ ID NO: 5, SEQ ID NO: 6) are given in Example (iv) of the instant invention, but also other primers generated from the sequences as disclosed in the instant invention can be used. A Northern blot or a ribonuclease protection assay (RPA) with probes specific for said gene can also be applied. It might further be preferred to measure transcription products by means of chip-based microarray technologies. These techniques are known to those of ordinary skill in the art (see Sambrook and Russell, Molecular Cloning: A Laboratory Manual, Cold Spring Harbor Laboratory Press, Cold Spring Harbor, New York, 2001; Schena M., Microarray Biochip Technology, Eaton Publishing, Natick, MA, 2000). An example of an immunoassay is the detection and measurement of enzyme activity as disclosed and described in the patent application WO 02/14543.

Furthermore, a level and/or an activity of a translation product of the gene coding for an ATP2B1 protein and/or of a fragment, or derivative, or variant of said translation product, and/or the level of activity of said translation product, and/or of a fragment, or derivative, or variant thereof, can be detected using an immunoassay, an activity assay, and/or a binding assay. These assays can measure the amount of binding between said protein molecule and an anti- protein antibody by the use of enzymatic, chromodynamic, radioactive, magnetic, or luminescent labels which are attached to either the anti- protein antibody or a secondary antibody which binds the anti- protein antibody. In addition, other high affinity ligands may be used. Immunoassays which can be used include e.g. ELISAs, Western blots and other techniques known to those of ordinary skill in the art (see Harlow and Lane, Antibodies: A Laboratory Manual, Cold Spring Harbor Laboratory Press, Cold Spring Harbor, New York, 1999 and Edwards R, Immunodiagnostics: A Practical Approach, Oxford University Press, Oxford; England, 1999) . All these detection techniques may also be employed in the format of microarrays, protein- arrays, antibody microarrays, tissue microarrays, electronic biochip or protein- chip based technologies (see Schena M., Microarray Biochip Technology, Eaton Publishing, Natick, MA, 2000) .

In a preferred embodiment, the level, expression or the activity, or both said level, expression and said activity of (i) a transcription product of the gene coding for an ATP2B1 protein, and/or of (ii) a translation product of the gene coding an ATP2B1 protein, in a series of samples taken from said subject over a period of time is compared, in order to monitor the progression of said disease. In further preferred embodiments, said subject receives a treatment prior to one or more of said sample gatherings. In yet another preferred embodiment, said level and/or activity is determined before and after said treatment of said subject.

In another aspect, the invention features the use of a kit in a method for diagnosing or prognosticating AD, in a subject, or for determining the predisposition of a subject to develop AD, or for monitoring the effect of a treatment administered to a subject having AD, said kit comprising:

at least one reagent which is selected from the group consisting of (i) reagents that detect a transcription product of the gene coding for ATP2B1 protein having SEQ ID NO:1 and/or for fragments, derivatives, variants thereof (ii) reagents that detect a translation product of the gene coding for ATP2B1 protein having SEQ ID NO:1. The kit may further comprise instructions for diagnosing, or prognosticating a neurodegenerative disease, in particular AD, and/or determining the propensity or predisposition of a subject to develop such a disease or of monitoring the effect of a treatment by:

- determine a level, expression or an activity, or both said level, expression and said activity, of said transcription product and/or said translation product of the gene coding for ATP2B1, in a sample obtained from said subject; and
- comparing said level and/or said activity and/or expression of said transcription product and/or said translation product to a reference value representing a known disease status (patient) and/or to a reference value representing a known health status (control) and/or to a reference value representing a known Braak stage; and
- analysing whether said level and/or said activity and/or expression is varied compared to a reference value representing a known health status, and/or is similar or equal to a reference value representing a known disease status or a reference value representing a known Braak stage; and
- diagnosing or prognosticating a neurodegenerative disease, in particular AD, or determining the propensity or predisposition of said subject to develop such a disease, wherein a varied or altered level, expression or activity, or both said level and said activity, of said transcription product and/or said translation product compared to a reference value representing a known health status (control) and/or wherein a level, expression or activity, or both said level and said activity, of said transcription product and/or said translation product is similar or equal to a reference value representing a known disease status (patient sample), preferably a disease status of AD (AD patient), and/or to a reference value representing a known Braak stage, indicates a diagnosis or prognosis of a neurodegenerative disease, in particular AD, or an increased propensity or predisposition of developing such a disease, a high risk of developing signs and symptoms of AD. The kit, according to the present invention, may be particularly useful for the identification of individuals that are at risk of developing AD.

Reagents that selectively detect a transcription product and/or a translation product of the gene coding for ATP2B1 protein can be sequences of various length, fragments of sequences, antibodies, aptamers, siRNA, microRNA, ribozymes.

Consequently, the kit, according to the present invention, may serve as a means for targeting identified individuals for early preventive measures or therapeutic intervention prior to disease onset, before irreversible damage in the course of the disease has been inflicted. Furthermore, in preferred embodiments, the kit featured in the invention is useful for monitoring a progression of a neurodegenerative disease, in particular AD in a subject, as well as monitoring success or failure of therapeutic treatment for such a disease of said subject.

Disclosed is a method of treating or preventing a neurodegenerative disease, in particular AD, in a subject comprising the administration to said subject in a therapeutically or prophylactically effective amount of an agent or agents which directly or indirectly affect a level, or an activity, or both said level and said activity, of (i) the gene coding for an ATP2B1 protein, and/or (ii) a transcription product of the gene coding for an ATP2B1 protein, and/or (iii) a translation product of the gene coding for an ATP2B1 protein, and/or (iv) a fragment, or derivative, or variant of (i) to (iii). Said agent may comprise a small molecule, or it may also comprise a peptide, an oligopeptide, or a polypeptide. Said peptide, oligopeptide, or polypeptide may comprise an amino acid sequence of a translation product of the gene coding for an ATP2B1 protein, or a fragment, or derivative, or a variant thereof. An agent for treating or preventing a neurodegenerative disease, in particular AD, according to the instant invention, may also consist of a nucleotide, an oligonucleotide, or a polynucleotide. Said oligonucleotide or polynucleotide may comprise a nucleotide sequence of the gene coding for an ATP2B1 protein, either in sense orientation or in antisense orientation.

In preferred embodiments, the method comprises the application of per se known methods of gene therapy and/or antisense nucleic acid technology to administer said agent or agents. In general, gene therapy includes several approaches: molecular replacement of a mutated gene, addition of a new gene resulting in the synthesis of a therapeutic protein, and modulation of endogenous cellular gene expression by recombinant expression methods or by drugs. Gene-transfer techniques are described in detail (see e.g. Behr, Acc Chem Res 1993, 26: 274-278 and Mulligan, Science 1993, 260: 926-931) and include direct gene-transfer techniques such as mechanical microinjection of DNA into a cell as well as indirect techniques employing biological vectors (like recombinant viruses, especially retroviruses) or model liposomes, or techniques based on transfection with DNA coprecipitation with polycations, cell membrane pertubation by chemical (solvents, detergents, polymers, enzymes) or physical means (mechanic, osmotic, thermic, electric shocks). The postnatal gene transfer into the central nervous system has been described in detail (see e.g. Wolff, Curr Opin Neurobiol 1993, 3: 743-748).

Additionally disclosed is a method of treating or preventing a neurodegenerative disease by means of antisense nucleic acid therapy, i.e. the down-regulation of an inappropriately expressed or defective gene by the introduction of antisense nucleic acids or derivatives thereof into certain critical cells (see e.g. Gillespie, DN&P 1992, 5: 389-395; Agrawal and Akhtar, Trends Biotechnol 1995, 13: 197-199; Crooke, Biotechnology 1992, 10: 882-6). Apart from hybridization strategies, the application of ribozymes, i.e. RNA molecules that act as enzymes, destroying RNA that carries the message of disease has also been described (see e.g. Barinaga, Science 1993, 262: 1512-1514). In preferred embodiments, the subject to be treated is a human, and therapeutic antisense nucleic acids or derivatives thereof are directed against transcription products of the gene coding for an ATP2B1 protein. It is preferred that cells of the central nervous system, preferably the brain, of a subject are treated in such a way. Cell penetration can be performed by known strategies such as coupling of antisense nucleic acids and derivatives thereof to carrier particles, or the above described techniques. Strategies for administering targeted therapeutic oligo-deoxynucleotides are known to those of skill in the art (see e.g. Wickstrom, Trends Biotechnol 1992, 10: 281-287). In some cases, delivery can be performed by mere topical application. Further approaches are directed to intracellular expression of antisense RNA. In this strategy, cells are transformed *ex vivo* with a recombinant gene that directs the synthesis of an RNA that is complementary to a region of target nucleic acid. Therapeutical use of intracellularly expressed antisense RNA is procedurally similar to gene therapy. A recently developed method of regulating the intracellular expression of genes by the use of double-stranded RNA, known variously as RNA interference (RNAi), can be another effective approach for nucleic acid therapy (Hannon, Nature 2002, 418: 244-251).

In further preferred embodiments, the method comprises grafting donor cells into the central nervous system, preferably the brain, of said subject, or donor cells preferably treated so as to minimize or reduce graft rejection, wherein said donor cells are genetically modified by insertion of at least one transgene encoding said agent or agents. Said transgene might be carried by a viral vector, in particular a retroviral vector. The transgene can be inserted into the donor cells by a nonviral physical transfection of DNA encoding a transgene, in particular by microinjection. Insertion of the transgene can also be performed by electroporation, chemically mediated transfection, in particular calcium phosphate transfection or liposomal mediated transfection (see Mc Cell and and Pardee, Expression Genetics: Accelerated and High-Throughput Methods, Eaton Publishing, Natick, MA, 1999).

In preferred embodiments, said agent for treating and preventing AD, is a therapeutic protein which can be administered to said subject, preferably a human, by a process comprising introducing subject cells into said subject, said subject cells having been treated *in vitro* to insert a DNA segment encoding said therapeutic protein, said subject cells expressing *in vivo* in said subject a therapeutically effective amount of said therapeutic protein. Said DNA segment can be inserted into said cells *in vitro* by a viral vector, in particular a retroviral vector.

Methods of treatment, according to the present invention, comprise the application of therapeutic cloning, transplantation, and stem cell therapy using neuronal adult stem cells, combined with any of the previously described cell- and gene therapeutic methods. Stem cells may be totipotent or pluripotent. They may also be organ-specific. Strategies for repairing diseased and/or damaged brain cells or tissue comprise (i) taking donor cells from an adult tissue. Use of differentiation factors then leads to a directed development of the stem cells to specialized cell types, preferably neuronal cells (Lanza et al., Nature Medicine 1999, 9: 975-977), or (ii) purifying adult stem cells, isolated from the central nervous system, or from bone marrow (mesenchymal stem cells), for in vitro expansion and subsequent grafting and transplantation, or (iii) directly inducing endogenous neural stem cells to proliferate, migrate, and differentiate into functional neurons (Peterson DA, Curr. Opin. Pharmacol. 2002, 2: 34-42). Adult neural stem cells are of great potential for repairing damaged or diseased brain tissues, as the germinal centers of the adult brain are free of neuronal damage or dysfunction (Colman A, Drug Discovery World 2001, 7: 66-71).

In preferred embodiments, the subject for treatment or prevention, according to the present invention, can be a human, an experimental non-human animal, e.g. a mouse or a rat, a domestic animal, or a non-human primate. The experimental non-human animal can be an animal model for a neurodegenerative disorder, e.g. a transgenic mouse and/or a knock-out mouse with an AD-type neuropathology.

Further disclosed is an agent, a selective antagonist or agonist or a modulator of an activity, or a level, or both said activity and said level, and/or of expression of at least one substance which is selected from the group consisting of (i) the gene coding for ATP2B1 protein, and/or (ii) a transcription product of the gene coding for ATP2B1 protein, and/or (iii) a translation product of the gene coding for ATP2B1 protein, and/or (iv) a fragment, or derivative, or variant of (i) to (iii), and said agent, selective antagonist or agonist, or said modulator has a potential activity in the treatment of neurodegenerative diseases, in particular AD.

In an additional aspect, the invention features a pharmaceutical composition comprising said modulator and preferably a pharmaceutical carrier. Said carrier refers to a diluent, adjuvant, excipient, or vehicle with which the modulator is administered.

Disclosed is a modulator of an activity, or a level,or expression or both said activity and said level of at least one substance which is selected from the group consisting of (i) the gene coding for an ATP2B1 protein, and/or (ii) a transcription product of the gene coding an ATP2B1 protein, and/or (iii) a translation product of the gene coding for an ATP2B1 protein, and/or (iv) a fragment, or derivative, or variant of (i) to (iii) for use in a pharmaceutical composition.

Disclosed is the use of an agent, an antibody, a selective antagonist or agonist, or a modulator of an activity, or a level, or both said activity and said level, and/or of expression of at least one substance which is selected from the group consisting of (i) the gene coding for ATP2B1 protein, and/or (ii) a transcription product of the gene coding for ATP2B1 protein, and/or (iii) a translation product of the gene coding for ATP2B1 protein, and/or (iv) a fragment, or derivative, or variant of (i) to (iii) in the manufacture of a medicament for treating or preventing a neurodegenerative disease, in particular AD. Said antibody may be specifically immunoreactive with an immunogen which is a translation product of a gene coding for ATP2B1 having SEQ ID NO: 1.

In one aspect, the present invention also provides a kit comprising one or more containers filled with a therapeutically or prophylactically effective amount of said pharmaceutical composition.

Further disclosed is a recombinant, genetically modified non-human animal comprising a non-native ATP2B1 gene sequence coding for a ATP2B1 protein having SEQ ID NO: 1, or a fragment, or a derivative, or variant thereof under the control of a transcriptional element which is not the native ATP2B1 gene transcriptional control element. The generation of said recombinant, non-human animal comprises (i) providing a gene targeting construct containing said gene sequence and a selectable marker sequence, and (ii) introducing said targeting construct into a stem cell of a non-human animal, and (iii) introducing said non-human animal stem cell into a non-human embryo, and (iv) transplanting said embryo into a pseudopregnant non-human animal, and (v) allowing said embryo to develop to term, and (vi) identifying a genetically altered non-human animal whose genome comprises a modification of said gene sequence in both alleles, and (vii) breeding the genetically altered non-human animal of step (vi) to obtain a genetically altered non-human animal whose genome comprises a modification of said endogenous gene, wherein said gene is mis-expressed, or under-expressed, or over-expressed, and wherein said disruption or alteration results in said non-human animal exhibiting a predisposition to developing signs and symptoms of a neurodegenerative disease, which signs and symptoms relate to AD. Strategies and techniques for the generation and construction of such an animal are known to those of ordinary skill in the art (see e.g. Capecchi, Science 1989, 244: 1288-1292 and Hogan et al., Manipulating the Mouse Embryo: A Laboratory Manual, Cold Spring Harbor Laboratory Press, Cold Spring Harbor, New York, 1994 and Jackson and Abbott, Mouse Genetics and Transgenics: A Practical Approach, Oxford University Press, Oxford, England, 1999). Also disclosed is the use of such a genetically modified, recombinant non-human animal as a test animal or as a control animal model for investigating neurodegenerative diseases, in particular Alzheimer's disease. Such an animal may be useful for screening, testing and validating compounds, agents and modulators in the development of diagnostics and therapeutics to treat neurodegenerative diseases, in particular Alzheimer's disease. The use of such a genetically modified animal in a screening method is disclosed in the instant invention.

In a further aspect the invention makes use of a cell, in which a gene sequence coding for a ATP2B1 protein having SEQ ID NO: 1, is mis-expressed, under-expressed, non-expressed or over-expressed, or disrupted or in another way altered for screening, testing and validating compounds, agents and modulators in the development of diagnostics and therapeutics to treat Alzheimer's disease. The use of such a cell in a screening method is disclosed in the instant invention.

In another aspect, the invention features a method of screening for identifying an agent, a modulator, a selective antagonist or agonist for use in the treatment of AD, or related diseases which agents, modulators or selective antagonists or agonists have an ability to alter expression or level or activity of one or more substances selected from the group consisting of (i) the gene coding for ATP2B1 protein having SEQ ID NO: 1, and/or (ii) a transcription product of the gene coding for ATP2B1 protein having SEQ ID NO: 1, and/or (iii) a translation product of the gene coding for ATP2B1 protein having SEQ ID NO: 1. This screening method comprises (a) contacting a cell with a test compound, and (b) measuring the activity and/or the level, or both the activity and the level, and/or the expression of one or more substances recited in (i) to (iii) and (c) measuring the activity and/or the level, or both the activity and the level and/or the expression of said substances in a control cell not contacted with said test compound, and comparing the levels and/or activities and/or the expression of the substance in the cells of step (b) and (c), wherein an alteration in the activity and/or level and/or expression of said substances in the contacted cells indicates that the test compound is an agent, modulator or selective antagonist or agonist for use in the treatment of Alzheimer's disease or related diseases. Said cells may be cells as disclosed in the instant invention.

In one further aspect, the invention features a method of screening for identifying an agent, a modulator, a selective antagonist or agonist for use in the treatment of AD, or related diseases, which agents, modulators or selective antagonists or agonists have an ability to alter expression or level or activity of one or more substances selected from the group consisting of (i) the gene coding for ATP2B1 protein having SEQ ID NO: 1, and/or (ii) a transcription product of the gene coding for ATP2B1 protein having SEQ ID NO: 1, and/or (iii) a translation product of the gene coding for ATP2B1 protein having SEQ ID NO: 1, comprising (a) administering a test compound to a non-human test animal which is predisposed to developing or has already developed signs and symptoms of a neurodegenerative disease or related diseases or disorders, and (b) measuring the activity and/or level and/or expression of one or more substances recited in (i) to (iii), and (c) measuring the activity and/or level and/or expression of said substances in a non-human control animal which is predisposed to developing or has already developed said signs and symptoms of a neurodegenerative disease or related diseases or disorders, and to which non-human animal no such test compound has been administered, and (d) comparing the activity and/or level and/or expression of the substances in the animals of step (b) and (c), wherein an alteration in the activity and/or level and/or expression of substances in the non-human test animal indicates that the test compound is an agent, modulator or selective antagonist or agonist for use in the treatment of Alzheimer's disease or related diseases.

In another embodiment, the present invention provides a method for producing a medicament comprising the steps of (i) identifying a modulator of Alzheimer's disease by a method of the aforementioned screening methods and (ii) admixing the modulator with a pharmaceutical carrier. However, said modulator may also be identifiable by other types of screening methods and assays.

Also disclosed is an assay for testing a compound, preferably for screening a plurality of compounds, for inhibition of binding between a ligand and ATP2B1 protein having SEQ ID NO: 1, or a fragment, or derivative, or variant thereof. Said screening assay comprises the steps of (i) adding a liquid suspension of said ATP2B1 protein having SEQ ID NO: 1, or a fragment, or derivative, or variant thereof, to a plurality of containers, and (ii) adding a compound or a plurality of compounds to be screened for said inhibition to said plurality of containers, and (iii) adding a detectable, preferably a fluorescently labelled ligand to said containers, and (iv) incubating said ATP2B1 protein, or said fragment, or derivative or variant thereof, and said compound or plurality of compounds, and said detectable, preferably fluorescently labelled ligand, and (v) measuring the amounts of preferably the fluorescence associated with said ATP2B1 protein, or with said fragment, or derivative, or variant thereof, and (vi) determining the degree of inhibition by one or more of said compounds of binding of said ligand to said ATP2B1 protein, or said fragment, or derivative, or variant thereof. It might be preferred to reconstitute said ATP2B1 translation product, or fragment, or derivative, or variant thereof into artificial liposomes to generate the corresponding proteoliposomes to determine the inhibition of binding between a ligand and said ATP2B1 translation product. Methods of reconstitution of ATP2B1 translation products from detergent into liposomes have been detailed (Schwarz et al., Biochemistry 1999, 38: 9456-9464; Krivosheev and Usanov, Biochemistry-Moscow 1997, 62: 1064-1073). Instead of utilizing a fluorescently labelled ligand, it might in some aspects be preferred to use any other detectable label known to the person skilled in the art, e.g. radioactive labels, and detect it accordingly. Said method may be useful for the identification of novel compounds as well as for evaluating compounds which have been improved or otherwise optimized in their ability to inhibit the binding of a ligand to a gene product of the gene coding for ATP2B1 protein, or a fragment, or derivative, or variant thereof. One example of a fluorescent binding assay, in this case based on the use of carrier particles, is disclosed and described in patent application WO 00/52451. A further example is the competitive assay method as described in patent WO 02/01226. Preferred signal detection methods for screening assays of the instant invention are described in the following patent applications: WO 96/13744, WO 98/16814, WO 98/23942, WO 99/17086, WO 99/34195, WO 00/66985, WO 01/59436, WO 01/59416.

In one further embodiment, the present invention provides a method for producing a medicament comprising the steps of (i) identifying a compound as an inhibitor of binding between a ligand and a gene product of the gene coding for ATP2B1 protein by the aforementioned inhibitory binding assay and (ii) admixing the compound with a pharmaceutical carrier. However, said compound may also be identifiable by other types of screening assays.

In another aspect, the invention features a method for testing a compound, preferably for screening a plurality of compounds to determine the binding of said compounds to ATP2B1 protein having SEQ ID NO: 1, or to a fragment, or derivative, or variant thereof. Said method comprises (i) adding a liquid suspension of said ATP2B1 protein, to a plurality of containers, and (ii) adding a detectable, preferably a fluorescently labelled compound or a plurality of detectable, preferably fluorescently labelled compounds to be screened for said binding to said plurality of containers, and (iii) incubating said ATP2B1 protein, and said detectable, preferably fluorescently labelled compound or detectable, preferably fluorescently labelled compounds, and (iv) measuring the amounts of preferably the fluorescence associated with said ATP2B1 protein, and (v) determining the binding by one or more of said compounds to said ATP2B1 protein. In this type of assay it might be preferred to use a fluorescent label. However, any other type of detectable label might also be employed. Also in this type of assay it might be preferred to reconstitute a ATP2B1 translation product into artificial liposomes as described in the present invention. Said assay methods may be useful for the identification of novel compounds as well as for evaluating compounds which have been improved or otherwise optimized in their ability to bind to ATP2B1 protein, or a fragment, or derivative, or variant thereof.

In one further embodiment, the present invention provides a method for producing a medicament comprising the steps of (i) identifying a compound as a binder to a gene product of the gene coding for ATP2B1 protein by the aforementioned binding assays and (ii) admixing the compound with a pharmaceutical carrier. However, said compound may also be identifiable by other types of screening assays.

In another embodiment, the present invention provides for a medicament obtainable by any of the methods according to the herein claimed screening assays. In one further embodiment, the instant invention provides for a medicament obtained by any of the methods according to the herein claimed screening assays.

The present invention features the use of protein molecules of SEQ ID NO: 1, said protein molecules being translation products of the gene coding for an ATP2B1, as diagnostic targets for detecting Alzheimer's disease.

The present invention further features the use of protein molecules of SEQ ID NO: 1, said protein molecules being translation products of the gene coding for an ATP2B1, as screening targets for reagents or compounds preventing, or treating, or ameliorating Alzheimer's disease.

The present invention features the used antibodies which are specifically immunoreactive with a protein molecule of the ATP2B1 having SEQ ID NO: 1, for detecting the pathological state of a cell in a sample obtained from a subject, comprising immunocytochemical staining of said cell with said antibody, wherein an altered degree of staining, or an altered staining pattern in said cell compared to a cell representing a known health status indicates a pathological state of said cell, which relates to AD. The immunogen may comprise immunogenic or antigenic epitopes or portions of a translation product of said gene, wherein said immunogenic or antigenic portion of a translation product is a polypeptide, and wherein said polypeptide elicits an antibody response in an animal, and wherein said polypeptide is immunospecifically bound by said antibody. Methods for generating antibodies are well known in the art (see Harlow et al., Antibodies, A Laboratory Manual, Cold Spring Harbor Laboratory Press, Cold Spring Harbor, New York, 1988) . The term "antibody", as employed in the present invention, encompasses all forms of antibodies known in the art, such as polyclonal, monoclonal, chimeric, recombinatorial, anti- idiotypic, humanized, or single chain antibodies, as well as fragments thereof (see Dubel and Breitling, Recombinant Antibodies, Wiley- Liss, New York, NY, 1999) . Antibodies of the present invention are useful, for instance, in a variety of diagnostic and therapeutic methods, based on state- in- the- art techniques (see Harlow and Lane, Using Antibodies: A Laboratory Manual, Cold Spring Harbor Laboratory Press, Cold Spring Harbor, New York, 1999 and Edwards R., Immunodiagnostics: A Practical Approach, Oxford University Press, Oxford, England, 1999) such as enzyme- immuno assays (e.g. enzyme- linked immunosorbent assay, ELISA), radioimmuno assays, chemoluminescence- immuno assays, Western- blot, immunoprecipitation and antibody microarrays. These methods involve the detection of translation products of an ATP2B1 gene, or fragments, or derivatives, or variants thereof.

Immunocytochemical staining of a cell can be carried out by a number of different experimental methods well known in the art. It might be preferred, however, to apply an automated method for the detection of antibody binding, wherein the determination of the degree of staining of a cell, or the determination of the cellular or subcellular staining pattern of a cell, or the topological distribution of an antigen on the cell surface or among organelles and other subcellular structures within the cell, are carried out according to the method described in US patent 6150173.

Other features and advantages of the invention will be apparent from the following description of figures and examples which are illustrative only and not intended to limit the remainder of the disclosure in any way.

FIGURES:

Figure 1 discloses the initial identification of the differential expression of the gene coding for an ATP2B1 protein, ATP2B1, in a fluorescence differential display screen. The figure shows a clipping of a large preparative fluorescent differential display gel. PCR products from the frontal cortex (F) and the temporal cortex (T) of two healthy control subjects and six AD patients were loaded in duplicate onto a denaturing polyacrylamide gel (from left to right). PCR products were obtained by amplification of the individual cDNAs with the corresponding one-base-anchor oligonucleotide and the specific Cy3 labelled random primers. The arrow indicates the migration position where significant differences in intensity of the signals for a transcription product of the ATP2B1 gene derived from frontal cortex and from the temporal cortex of AD patients as compared to healthy controls exist. The differential expression reflects a down-regulation of human ATP2B1 gene transcription in the temporal cortex compared to the frontal cortex of AD patients and compared to the temporal cortex of healthy control subjects. Comparing the signals derived from temporal cortex and frontal cortex of healthy non-AD control subjects with each other, no difference in signal intensity, i.e. no altered expression level can be detected.

Figures 2 and 3 illustrate the verification of the differential expression of the ATP2B1 gene coding for ATP2B1 in AD brain tissues by quantitative RT-PCR analysis. Quantification of RT-PCR products from RNA samples collected from the frontal cortex (F) and the temporal cortex (T) of AD patients (Figure 2a) and samples from the frontal cortex (F) and the hippocampus (H) of AD patients (Figure 3a) was performed by the LightCycler rapid thermal cycling technique. Likewise, samples of healthy, age-matched control individuals were compared (Figure 2b for frontal cortex and temporal cortex, Figure 3b for frontal cortex and hippocampus). The data were normalized to the combined average values of a set of standard genes which showed no significant differences in their gene expression levels. Said set of standard genes consisted of genes for cyclophilin B, the ribosomal protein S9, the transferrin receptor, GAPDH, and beta-actin. The figures depict the kinetics of amplification by plotting the cycle number against the amount of amplified material as measured by its fluorescence. Note that the amplification kinetics of ATP2B1 cDNAs from both, the frontal and temporal cortices of a normal control individual, and from the frontal cortex and hippocampus of a normal control individual, respectively, during the exponential phase of the reaction are juxtaposed (Figures 2b and 3b, arrowhead), whereas in Alzheimer's disease (Figures 2a and 3a, arrowhead) there is a significant separation of the corresponding curves, indicating a differential expression of the gene ATP2B1 coding for ATP2B1 in the respective analyzed brain regions, indicating a dysregulation, preferably a downregulation of a transcription product of the human ATP2B1 gene, or a fragment, or derivative, or variant thereof, in the temporal cortex relative to the frontal cortex and in the hippocampus relative to the frontal cortex, respectively.

Figure 4 shows the analysis of absolute mRNA expression of ATP2B1 by comparison of control and AD stages using statistical method of the median at 98%-confidence level. The data were calculated by defining control groups including subjects with either Braak stages 0 to 1, Braak stages 0 to 2, or Braak stages 0 to 3 which are compared with the data calculated for the defined AD patient groups including Braak stages 2 to 6, Braak stages 3 to 6 and Braak stages 4 to 6, respectively. Additionally, three groups including subjects with either Braak stages 0 to 1, Braak stages 2 to 3 and Braak stages 4 to 6, respectively, were compared with each other. A significant difference was detected comparing frontal cortex (F) and inferior temporal cortex (T) of AD patients and of control persons with each other. Said difference reflects a down-regulation of ATP2B1 in the temporal cortex and frontal cortex of AD patients relative to the temporal cortex and frontal cortex of control persons and a down-regulation of ATP2B1 in the temporal cortex of AD patients compared to their frontal cortices. Said significant differences were observed already at Braak stage 3.

Figure 5 discloses SEQ ID NO: 1, the amino acid sequence of the ATP2B protein ATP2B1. The full length human ATP2B1 protein comprises 1176 amino acids.

Figure 6 shows SEQ ID NO: 2, the nucleotide sequence of the human ATP2B1 cDNA, constructed from Genome Database mRNAs and ESTs (see Figure 10), comprising 5178 nucleotides.

Figure 7 depicts SEQ ID NO: 3, the nucleotide sequence of the 644 bp ATP2B1 cDNA fragment, identified and obtained by differential display and subsequent cloning (sequence in 5' to 3' direction).

Figure 8 shows the nucleotide sequence of SEQ ID NO: 4, the coding sequence (cds) of the human ATP2B1 gene, comprising 3531 nucleotides, harbouring nucleotides 182 to 3712 of SEQ ID NO: 2.

Figure 9 outlines the sequence alignment over 640 base pairs of SEQ ID NO: 3 to the nucleotide sequence of the human ATP2B1 cDNA, SEQ ID NO: 2.

Figure 10 schematically charts the assembly of SEQ ID NO: 2 and of the coding sequence (cds) of ATP2B1 from genomic database sequence fragments, constituting the ATP2B1 encoding consensus cDNA sequence, a prolongated and corrected consensus sequence based on and derived from Genbank mRNAs and EST sequence fragments. The corresponding accession numbers are indicated on the left side.

Figure 11 schematically charts the alignment of the cDNA sequence encoding ATP2B1, SEQ ID NO: 2, the identified cDNA fragment sequence SEQ ID NO: 3, both primer sequences used for ATP2B1 transcription level profiling (primer A, primer B) and the coding sequence (cds) of ATP2B1, SEQ ID NO: 4. The sequence positions are indicated on the right side.

Figure 12 lists ATP2B1 gene expression levels in the temporal cortex relative to the frontal cortex in fifteen AD patients, herein identified by internal reference numbers P010, P011, P012, P014, P016, P017, P019, P038, P040, P041, P042, P046, P047, P048, P049 and twentyfive age-matched control individuals, herein identified by internal reference numbers C005, C008, C011, C012, C014, C025, C026, C027, C028, C029, C030, C031, C032, C033, C034, C035, C036, C038, C039, C041, C042, DE02, DE03, DE05, DE07. For an up-regulation in the temporal cortex, the values shown are calculated according to the formula described herein (see below) and in case of an up-regulation in the frontal cortex the reciprocal values are calculated, respectively. The bar diagram visualizes individual natural logarithmic values of the temporal to frontal cortex, ln(IT/IF), and of the frontal to temporal cortex regulation factors, ln(IF/IT), in different Braak stages (0 to 6).

Figure 13 lists the gene expression levels in the hippocampus relative to the frontal cortex for the ATP2B gene coding for ATP2B1 in six Alzheimer's disease patients, herein identified by internal reference numbers P010, P011, P012, P014, P016, P019 (0.16 to 0.70 fold) and two healthy, age-matched control individuals, herein identified by internal reference numbers C004, C008 (0.58 to 0.80 fold). The values shown are calculated according to the formula described herein (see below). The scatter diagram visualizes individual logarithmic values of the hippocampus to frontal cortex regulation ratios, log (ratio HC/IF), in control samples (dots) and in AD patient samples (triangles).

Figure 14 exemplarily depicts micrographs digitally taken from sections of the inferior temporal gyrus from a control donor (Control T) and from an Alzheimer patient (Patient T) immunolabeled with goat polyclonal anti- ATP2B1 (anti-PMCA1) antiserum (green signals) (magnification 10x) . Overall immunoreactivity of ATP2B1 was observed in the cerebral cortex (CT) and in the white matter (WM) . Figures 14A and 14E display the immunoreactivity of neurons (neuronal nuclei and somata) stained with an antibody against the neuron- specific marker NeuN. Figures 14B and 14F show the signals resulting from immunostaining with the anti- ATP2B1 goat polyclonal antiserum. Figures 14C and 14G each display a picture which results from merging the pictures 14A, 14B and DAPI staining and the pictures 14E, 14F and DAPI staining, respectively, wherein the colors code for the detected immunosignals from NeuN (red), ATP2B1 (green) and for the nuclear staining with DAPI (blue) . Immunoreactivity of ATP2B1 was detected in most neurons (cytoplasm, plasma membranes), as well weakly in astrocytes and in some oligodendrocytes but not in microglial cells. The data exemplarily shown here clearly indicate that the level of intensity and quantity of neuronal immunoreactivity of the ATP2B1 protein is reduced in the inferior temporal cortex from patients (Figures 14G and 14F) as compared to the inferior temporal cortex from control persons (Figures 14C and 14B) and as compared to both the frontal cortex from patients and from controls (see Figures 15G, 15F and Figures 15C, 15B) . Additionally, in comparison with sections from controls, astrocytic ATP2B1 immunoreactivity is increased in both the frontal and temporal specimens from patients.

Figure 15 exemplarily depicts micrographs digitally taken from sections of the frontal cortex from a control donor (Control F) and from an Alzheimer patient (Patient F) immunolabeled with goat polyclonal anti- ATP2B1 (anti-PMCA1) antiserum (green signals) (magnification 10x) . Overall immunoreactivity of ATP2B1 was observed in the cerebral cortex (CT) and in the white matter (WM) . Figures 15A and 15E display the immunoreactivity of neurons (neuronal nuclei and somata) stained with an antibody against the neuron- specific marker NeuN. Figures 15B and 15F show the signals resulting from immunostaining with the anti- ATP2B1 goat polyclonal antiserum. Figures 15C and 15G each display a picture which results from merging the pictures 15A, 15B and DAPI staining and the pictures 15E, 15F and DAPI staining, respectively, wherein the colors code for the detected immunosignals from NeuN (red), ATP2B1 (green) and for the nuclear staining with DAPI (blue) . Immunoreactivity of ATP2B1 was detected in most neurons (cytoplasm, plasma membranes), as well weakly in astrocytes and in some oligodendrocytes but not in microglial cells. The data exemplarily shown here clearly indicate that the level of intensity and quantity of neuronal immunoreactivity of the ATP2B1 protein is equal or similar in the frontal cortex from patients (Figures 15G and 15F) as compared to the frontal cortex from control persons (Figures 15C and 15B) but is higher in the frontal cortex from controls as well as from patients as compared to the temporal cortex from patients (see Figures 14G, 14F) . Additionally, in comparison with sections from controls, astrocytic ATP2B1 immunoreactivity increased in both the frontal and temporal specimens from patients.

EXAMPLE I:

(i) Brain tissue dissection from patients with AD:

Brain tissues from AD patients and age-matched control subjects were collected, on average, within 6 hours post-mortem and immediately frozen on dry ice. Sample sections from each tissue were fixed in paraformaldehyde for histopathological confirmation of the diagnosis. Brain areas for differential expression analysis were identified and stored at -80 °C until RNA extractions were performed.

(ii) Isolation of total mRNA:

Total RNA was extracted from post-mortem brain tissue by using the RNeasy kit (Qiagen) according to the manufacturer's protocol. The accurate RNA concentration and the RNA quality were determined with the DNA LabChip system using the Agilent 2100 Bioanalyzer (Agilent Technologies). For additional quality testing of the prepared RNA, i.e. exclusion of partial degradation and testing for DNA contamination, specifically designed intronic GAPDH oligonucleotides and genomic DNA as reference control were utilised to generate a melting curve with the LightCycler technology as described in the supplied protocol by the manufacturer (Roche).

(iii) cDNA synthesis and identification of differentially expressed genes by fluorescence differential display (FDD):

In order to identify changes in gene expression in different tissue, a modified and improved differential display (DD) screening method was employed. The original DD screening method is known to those skilled in the art (Liang and Pardee, Science 1995, 267:1186-7). This technique compares two populations of RNA and provides clones of genes that are expressed in one population but not in the other. Several samples can be analyzed simultaneously and both up- and downregulated genes can be identified in the same experiment. By adjusting and refining several steps in the DD method as well as modifying technical parameters, e.g. increasing redundancy, evaluating optimized reagents and conditions for reverse transcription of total RNA, optimizing polymerase chain reactions (PCR) and separation of the products thereof, a technique was developed which allows for highly reproducible and sensitive results. The applied and improved DD technique was described in detail by von der Kammer et al. (Nucleic Acids Research 1999, 27: 2211-2218). A set of 64 specifically designed random primers were developed (standard set) to achieve a statistically comprehensive analysis of all possible RNA species. Further, the method was modified to generate a preparative DD slab-gel technique, based on the use of fluorescently labelled primers. In the present invention, RNA populations from carefully selected post-mortem brain tissues (frontal and temporal cortex) of Alzheimer's disease patients and age-matched control subjects were compared. As starting material for the DD analysis we used total RNA, extracted as described above (ii). Equal amounts of 0.05 μg RNA each were transcribed into cDNA in 20 μl reactions containing 0.5 mM each dNTP, 1 μ Sensiscript Reverse Transcriptase and 1x RT buffer (Qiagen), 10 U RNase inhibitor (Qiagen) and 1 μM of either one-base-anchor oligonucleotides HT11A, HT11G or HT11C (Liang et al., Nucleic Acids Research 1994, 22: 5763-5764; Zhao et al., Biotechniques 1995, 18: 842-850). Reverse transcription was performed for 60 min at 37°C with a final denaturation step at 93°C for 5 min. 2 μl of the obtained cDNA each was subjected to a polymerase chain reaction (PCR) employing the corresponding one-base-anchor oligonucleotide (1 μM) along with either one of the Cy3 labelled random DD primers (1 μM), 1x GeneAmp PCR buffer (Applied Biosystems), 1.5 mM MgCl2 (Applied Biosystems), 2 μM dNTP-Mix (dATP, dGTP, dCTP, dTTP Amersham Pharmacia Biotech), 5 % DMSO (Sigma), 1 U AmpliTaq DNA Polymerase (Applied Biosystems) in a 20 μl final volume. PCR conditions were set as follows: one round at 94°C for 30 sec for denaturing, cooling 1°C/sec down to 40°C, 40°C for 4 min for low-stringency annealing of primer, heating 1°C/sec up to 72°C, 72°C for 1 min for extension. This round was followed by 39 high-stringency cycles: 94°C for 30 sec, cooling 1°C/sec down to 60°C, 60°C for 2 min, heating 1°C/sec up to 72°C, 72°C for 1 min. One final step at 72°C for 5 min was added to the last cycle (PCR cycler: Multi Cycler PTC 200, MJ Research). 8 μl DNA loading buffer were added to the 20 μl PCR product preparation, denatured for 5 min and kept on ice until loading onto a gel. 3.5 μl each were separated on 0.4 mm thick, 6% polyacrylamide (Long Ranger)/ 7 M urea sequencing gels in a slab-gel system (Hitachi Genetic Systems) at 2000 V, 60W, 30 mA, for 1 h 40 min. Following completion of the electrophoresis, gels were scanned with a FMBIO II fluorescence-scanner (Hitachi Genetic Systems), using the appropriate FMBIO II Analysis 8.0 software. A full-scale picture was printed, differentially expressed bands marked, excised from the gel, transferred into 1.5 ml containers, overlayed with 200 μl sterile water and kept at -20°C until extraction.

Elution and reamplification of DD products: The differential bands were extracted from the gel by boiling in 200 μl H2O for 10 min, cooling down on ice and precipitation from the supernatant fluids by using ethanol (Merck) and glycogen/sodium acetate (Merck) at -20°C over night, and subsequent centrifugation at 13.000 rpm for 25 min at 4°C. Pellets were washed twice in ice-cold ethanol (80%), resuspended in 10 mM Tris pH 8.3 (Merck) and dialysed against 10 % glycerol (Merck) for 1 h at room temperature on a 0.025 μm VSWP membrane (Millipore). The obtained preparations were used as templates for reamplification by 15 high-stringency cycles in 25-μl PCR mixtures containing the corresponding primer pairs as used for the DD PCR (see above) under identical conditions, with the exception of the initial round at 94°C for 5 min, followed by 15 cycles of: 94°C for 45 sec, 60°C for 45 sec, ramp 1°C/sec to 70°C for 45 sec, and one final step at 72°C for 5 min.

Cloning and sequencing of DD products: Re-amplified cDNAs were analyzed with the DNA LabChip system (Agilent 2100 Bioanalyzer, Agilent Technologies) and ligated into the pCR-Blunt II-TOPO vector and transformed into *E.coli* Top10F' cells (Zero Blunt TOPO PCR Cloning Kit, Invitrogen) according to the manufacturer's instructions. Cloned cDNA fragments were sequenced by commercially available sequencing facilities. The result of one such FDD experiment for the ATP2B gene coding for ATP2B1 protein is shown in Figure 1.

(iv) Confirmation of differential expression by quantitative RT-PCR:

Positive corroboration of differential ATP2B1 gene expression in the temporal cortex versus frontal cortex and in the hippocampus versus frontal cortex was performed using the LightCycler technology (Roche). This technique features rapid thermal cyling for the polymerase chain reaction as well as real-time measurement of fluorescent signals during amplification and therefore allows for highly accurate quantification of RT-PCR products by using a kinetic, rather than an endpoint readout. The ratios of ATP2B1 cDNAs from the temporal cortices of AD patients and of healthy age-matched control individuals, from the frontal cortices of AD patients and of healthy age-matched control individuals, from the hippocampi of AD patients and of healthy age-matched control individuals and the ratios of ATP2B1 cDNAs from the temporal cortex and frontal cortex of AD patients and of healthy age-matched control individuals, and the ratios of ATP2B1 cDNAs from the hippocampus and frontal cortex of AD patients and of healthy age-matched control individuals, respectively, were determined (relative quantification).

The mRNA expression profiling between frontal cortex tissue (F) and inferior temporal cortex tissue (T) of ATP2B1 has been analyzed in four up to nine tissues per Braak stage. Because of the lack of high quality tissues from one donor with Braak 3 pathology, tissues of one additional donor with Braak 2 pathology were included, and because of the lack of high quality tissues from one donor with Braak 6 pathology, tissue samples of one additional donor with Braak 5 pathology were included.

For the analysis of the profiling two general approaches have been applied. Both comparative profiling studies, frontal cortex against inferior temporal cortex as well as control against AD patients, which contribute to the complex view of the relevance of ATP2B, of ATP2B1 respectively, in AD physiology, are shown in detail below.

1) Relative comparison of the mRNA expression between frontal cortex tissue and inferior temporal cortex tissue of controls and of AD patients, respectively. This approach allowed to verify that the identified gene ATP2B1 is either involved in the protection of the less vulnerable tissue (frontal cortex) against degeneration, or is involved in or enhances the process of degeneration in the more vulnerable tissue (inferior temporal cortex).

First, a standard curve was generated to determine the efficiency of the PCR with specific primers for the gene coding for ATP2B1:

Primer A, SEQ ID NO: 5, 5'-GATCACGCTGAAAGGGAGTTG-3' and Primer B, SEQ ID NO: 6, 5'-TTAGAGCCCCCTGAATGGAAC-3'.

PCR amplification (95°C and 1 sec, 56°C and 5 sec, and 72°C and 5 sec) was performed in a volume of 20 μl containing LightCycler-FastStart DNA Master SYBR Green I mix (contains FastStart Taq DNA polymerase, reaction buffer, dNTP mix with dUTP instead of dTTP, SYBR Green I dye, and 1 mM $MgCl_2$; Roche), 0.5 μM primers, 2 μl of a cDNA dilution series (final concentration of 40, 20, 10, 5, 1 and 0.5 ng human total brain cDNA; Clontech) and, depending on the primers used, additional 3 mM Mac12- Melting curve analysis revealed a single peak at approximately 84.3°C with no visible primer dimers. Quality and size of the PCR product were determined with the DNA LabChip system (Agilent 2100 Bioanalyzer, Agilent Technologies). A single peak at the expected size of 124 bp for the gene coding for ATP2B1 protein was observed in the electropherogram of the sample.

In an analogous manner, the PCR protocol was applied to determine the PCR efficiency of a set of reference genes which were selected as a reference standard for quantification. In the present invention, the mean value of five such reference genes was determined: (1) cyclophilin B, using the specific primers SEQ ID NO: 7, 5'-ACTGAAGCACTACGGGCCTG-3' and SEQ ID NO: 8, 5'-AGCCGTTGGTGTCTTTGCC-3' except for $MgCl_2$ (an additional 1 mM was added instead of 3 mM). Melting curve analysis revealed a single peak at approximately 87°C with no visible primer dimers. Agarose gel analysis of the PCR product showed one single band of the expected size (62 bp). (2) Ribosomal protein S9 (RPS9), using the specific primers SEQ ID NO: 9, 5'-GGTCAAATTTACCCTGGCCA-3' and SEQ ID NO: 10, 5'- TCTCATCAAGCGTCAGCAGTTC-3' (exception: additional 1 mM $MgCl_2$ was added instead of 3 mM). Melting curve analysis revealed a single peak at approximately 85°C with no visible primer dimers. Agarose gel analysis of the PCR product showed one single band with the expected size (62 bp). (3) beta-actin, using the specific primers SEQ ID NO: 11, 5'-TGGAACGGTGAAGGTGACA-3' and SEQ ID NO: 12, 5'-GGCAAGGGACTTCCTGTAA-3'. Melting curve analysis revealed a single peak at approximately 87°C with no visible primer dimers. Agarose gel analysis of the PCR product showed one single band with the expected size (142 bp). (4) GAPDH, using the specific primers SEQ ID NO: 13, 5'-CGTCATGGGTGTGAACCATG-3' and SEQ ID NO: 14, 5'-GCTAAGCAGTTGGTGGTGCAG-3'. Melting curve analysis revealed a single peak at approximately 83°C with no visible primer dimers. Agarose gel analysis of the PCR product showed one single band with the expected size (81 bp). (5) Transferrin receptor TRR, using the specific primers SEQ ID NO: 15, 5'-GTCGCTGGTCAGTTCGTGATT-3' and SEQ ID NO: 16, 5'-AGCAGTTGGCTGTTGTACCTCTC-3'. Melting curve analysis revealed a single peak at approximately 83°C with no visible primer dimers. Agarose gel analysis of the PCR product showed one single band with the expected size (80 bp).

For calculation of the values, first the logarithm of the cDNA concentration was plotted against the threshold cycle number $C_t$ for the gene coding for ATP2B1 protein and the five reference standard genes. The slopes and the intercepts of the standard curves (i.e. linear regressions) were calculated for all genes. In a second step, cDNAs from frontal cortices of AD patients and of healthy control individuals, from temporal cortices of AD patients and of healthy control individuals, from hippocampi of AD patients and of healthy control individuals, and cDNAs from the frontal cortex and the temporal cortex of AD patients and of control individuals and from the frontal cortex and the hippocampus of AD patients and of control individuals, respectively, were analyzed in parallel and normalized to cyclophilin B. The $C_t$ values were measured and converted to ng total brain cDNA using the corresponding standard curves:

$$10 \wedge ( (C_t \text{ value - intercept}) / \text{slope} ) \qquad [\text{ng total brain cDNA}]$$

The values for temporal and frontal cortex ATP2B1 cDNAs, the values for hippocampus and frontal cortex ATP2B1 cDNAs, and the values from the frontal cortex ATP2B1 cDNAs of AD patients (P) and control individuals (C), and the values for temporal cortex ATP2B1 cDNAs of AD patients (P) and of control individuals (C), respectively, were normalized to cyclophilin B and the ratios were calculated according to formulas:

$$\text{Ratio} = \frac{\text{ATP2B1 temporal [ng] / cyclophilin B temporal [ng]}}{\text{ATP2B1 frontal [ng] / cyclophilin B frontal [ng]}}$$

$$\text{Ratio} = \frac{\text{ATP2B1 hippocampus [ng] / cyclophilin B hippocampus [ng]}}{\text{ATP2B1 frontal [ng] / cyclophilin B frontal [ng]}}$$

$$\text{Ratio} = \frac{\text{ATP2B1 P temporal [ng] / cyclophilin B P temporal [ng]}}{\text{ATP2B1 C temporal [ng] / cyclophilin B C temporal [ng]}}$$

$$\text{Ratio} = \frac{\text{ATP2B1 P frontal [ng] / cyclophilin B P frontal [ng]}}{\text{ATP2B1 C frontal [ng] / cyclophilin B C frontal [ng]}}$$

In a third step, the set of reference standard genes was analyzed in parallel to determine the mean average value of the AD patient to control person temporal cortex ratios, of the AD patient to control person frontal cortex ratios, and of the temporal to frontal ratios of AD patients and control persons and the hippocampi to frontal ratios of AD patients and control persons, respectively, of expression levels of the reference standard genes for each individual brain sample. As cyclophilin B was analyzed in step 2 and step 3, and the ratio from one gene to another gene remained constant in different runs, it was possible to normalize the values for the gene coding for ATP2B1 protein to the mean average value of the set of reference standard genes instead of normalizing to one single gene alone. The calculation was performed by dividing the respective ratio shown above by the deviation of cyclophilin B from the mean value of all housekeeping genes. The results of such quantitative RT-PCR analysis for the gene coding for ATP2B1 protein are shown in Figures 2, 3 and 12, 13. 2) Comparison of the mRNA expression between controls and AD patients.

For this analysis it was proven that absolute values of real-time quantitative PCR (Lightcycler method) between different experiments at different time points are consistent enough to be used for quantitive comparisons without usage of calibrators. Cyclophilin was used as a standard for normalization in any of the qPCR experiments for more than 100 tissues. Between others it was found to be the most consistently expressed housekeeping gene in our normalization experiments. Therefore a proof of concept was done by using values that were generated for cyclophilin.

First analysis used cyclophilin values from qPCR experiments of frontal cortex and inferior temporal cortex tissues from three different donors. From each tissue the same cDNA preparation was used in all analyzed experiments. Within this analysis no normal distribution of values was achieved due to small number of data. Therefore the method of median and its 98 %-conficence level was applied. This analysis revealed a middle deviation of 8.7 % from the median for comparison of absolute values and a middle deviation of 6.6 % from the median for relative comparison.

Second analysis used cyclophilin values from qPCR experiments of frontal cortex and inferior temporal cortex tissues from two different donors each, but different cDNA preparations from different time points were used. This analysis revealed a middle deviation of 29.2 % from the median for comparison of absolute values and a middle deviation of 17.6 % from the median for relative comparison. From this analysis it was concluded, that absolute values from qPCR experiments can be used, but the middle deviation from median should be taken into further considerations. A detailed analysis of absolute values for ATP2B1 was performed. Therefore, absolute levels of ATP2B1 were used after relative normalization with cyclophilin. The median as well as the 98 %-confidence level was calculated for the control group (Braak 0 - Braak 3) and the patient group (Braak 4 - Braak 6), respectively. Same analysis was done redefining the control group (Braak 0 - Braak 2) and the patient group (Braak 3 - Braak 6) as well as redefining the control group (Braak 0 - Braak 1) and the patient group (Braak 2 - Braak 6). The latter analysis was aimed to identify early onset of mRNA expression differences between controls and AD patients. In another view of this analysis, three groups comprising Braak stages 0-1, Braak stages 2-3, and Braak stages 4-6, respectively, were compared to each other in order to identify tendencies of gene expression regulation as well as early onset differences. Said analysis described above is shown in Figure 4.

(v) Immunohistochemistry:

For immunofluorescence staining of ATP2B1 in human brain, post- mortem fresh- frozen frontal and temporal forebrain specimens from donors comprising patients with clinically diagnosed and neuropathologically confirmed Alzheimer's disease at various Braak stages as well as age- matched control individuals without Alzheimer, were cut at 14 μm thickness using a cryostat (Leica CM3050S) . The tissue sections were air- dried at room temprature and fixed in acetone for 10 min. After washing in PBS, the sections were pre- incubated with blocking buffer (10% normal horse serum, 0.2% Triton X- 100 in PBS) for 30 min and then incubated with anti- ATP2B1 goat polyclonal antiserum (1: 8 diluted in blocking buffer; Santa Cruz, sc- 16488) overnight at 4°C. After rinsing three times in 0.1% Triton X- 100/PBS, the sections were incubated with FITC- conjugated donkey anti- goat IgG antiserum (Dianova, 705- 096- 147, 1: 150 diluted in 1% BSA/PBS) for 2 hours at room temperature and then again washed in PBS. Staining of the neuronal cells was performed as described above using a mouse monoclonal antibody against the neuronal specific marker NeuN (Chemicon, MAB377, dilution 1: 200) and a secondary Cy3- conjugated donkey anti- mouse antibody (Dianova, 115- 166- 150, dilution 1: 600) . Staining of the nuclei was performed by incubation of the sections with 5μM DAPI in PBS for 3 min. In order to block the autofluoresence of lipofuscin in human brain, the sections were treated with 1% Sudan Black B in 70% ethanol for 2- 10 min at room temperature and then sequentially dipped in 70% ethanol, destilled water and PBS. The sections were coverslipped with 'Vectashield' mounting medium (Vector Laboratories, Burlingame, CA) . Microscopic images were obtained using dark field epifluorescence and bright field phase contrast illumination conditions (IX81, Olympus Optical) . Microscopic images were digitally captured with a PCO SensiCam and analyzed using the appropriate software (AnalySiS, Olympus Optical) (see Figures 14 and 15) .

## Claims

1. A method of diagnosing or prognosticating Alzheimer's disease in a subject, or determining whether a subject is at increased risk of developing said disease, or of monitoring the effect of a treatment administered to a subject having said disease, comprising determining a level and/or an activity of

   (i) a transcription product of the gene coding for a plasma membrane calcium ATPase having SEQ ID NO: 1, and/or
   (ii) a translation product of the gene coding for a plasma membrane calcium ATPase having SEQ ID NO: 1,

   in a sample obtained from said subject and comparing said level and/or said activity to a reference value representing a known disease status and/or to a reference value representing a known health status, and said level and/or said activity is varied compared to a reference value representing a known health status, and/or is similar or equal to a reference value representing a known disease status, thereby diagnosing or prognosticating Alzheimer's disease in said subject, or determining whether said subject is at increased risk of developing said disease.

2. Use of a kit in a method according to claim 1 for diagnosing or prognosticating Alzheimer's disease, or for determining the predisposition of a subject to develop such a disease, or for monitoring the effect of a treatment administered to a subject having Alzheimer's disease, said kit comprising at least one reagent which is selected from the group consisting of (i) reagents that detect a transcription product of a gene coding for a plasma membrane calcium ATPase having SEQ ID NO: 1 and/or (ii) reagents that detect a translation product of the gene coding for a plasma membrane calcium ATPase having SEQ ID NO: 1.

3. A method of screening for identifying agents, modulators or selective antagonists or agonists for use in the treatment of Alzheimer's disease or related diseases, which agents, modulators or selective antagonists or agonists have an ability to alter expression or level or activity of one or more substances selected from the group consisting of

   (i) a gene coding for a plasma membrane calcium ATPase having SEQ ID NO: 1, and/or
   (ii) a transcription product of the gene coding for a plasma membrane calcium ATPase having SEQ ID NO: 1, and/or
   (iii) a translation product of the gene coding for a plasma membrane calcium ATPase having SEQ ID NO: 1,
   said method comprises:

      (a) contacting a cell with a test compound;
      (b) measuring the activity and/or level and/or expression of one or more substances recited in (i) to (iii);
      (c) measuring the activity and/or level and/or expression of one or more substances recited in (i) to (iii) in a control cell not contacted with said test compound; and comparing the levels and/or activities and/or expression of the substances in the cells of step (b) and (c), wherein an alteration in the activity and/or level and/or expression of the substances in the contacted cells indicates that the test compound is an agent, modulator or selective antagonist or agonist for use in the treatment of Alzheimer's disease or related diseases.

**4.** A method of screening for identifying agents, modulators or selective antagonists or agonists for use in the treatment of Alzheimer's disease or related diseases, which agents, modulators or selective antagonists or agonists have an ability to alter expression or level or activity of one or more substances selected from the group consisting of

(i) a gene coding for a plasma membrane calcium ATPase having SEQ ID NO: 1, and/or
(ii) a transcription product of the gene coding for a plasma membrane calcium ATPase having SEQ ID NO: 1, and/or
(iii) a translation product of the gene coding for a plasma membrane calcium ATPase having SEQ ID NO: 1,

said method comprises:

(a) administering a test compound to a non-human test animal which is predisposed to developing or has already developed signs and symptoms of a neurodegenerative disease or related diseases or disorders;
(b) measuring the activity and/or level and/or expression of one or more substances recited in (i) to (iii);
(c) measuring the activity and/or level and/or expression of one or more substances recited in (i) or (iii) in a non-human control animal which is predisposed to developing or has already developed signs and symptoms of a neurodegenerative disease or related diseases or disorders and to which non-human animal no such test compound has been administered;
(d) comparing the activity and/or level and/or expression of the substances in the animals of step (b) and (c), wherein an alteration in the activity and/or level and/or expression of substances in the non-human test animal indicates that the test compound is an agent, modulator or selective antagonist or agonist for use in the treatment of Alzheimer's disease or related diseases.

**5.** The method according to claim 4 wherein said non-human test animal and/or said non-human control animal is a recombinant animal which expresses a plasma membrane calcium ATPase having SEQ ID NO: 1, under the control of a transcriptional control element which is not the native plasma membrane calcium ATPase gene transcriptional control element.

**6.** A method for testing a compound, preferably for screening a plurality of compounds to determine the binding of said compounds to a plasma membrane calcium ATPase having SEQ ID NO: 1, said method comprising the steps of:

(i) adding a liquid suspension of said plasma membrane calcium ATPase, or a fragment, or derivative, or variant thereof, to a plurality of containers;
(ii) adding a detectable, in particular a fluorescently labelled compound or a plurality of detectable, in particular fluorescently labelled compounds to be screened for said binding to said plurality of containers;
(iii) incubating said plasma membrane calcium ATPase, and said detectable, in particular fluorescently labelled compound or detectable, in particular fluorescently labelled compounds;
(iv) measuring amounts of preferably the fluorescence associated with said plasma membrane calcium ATPase; and
(v) determine the binding by one or more of said compounds to said plasma membrane calcium ATPase.

**7.** Use of protein molecules of SEQ ID NO: 1, said protein molecules being translation products of the gene coding for a plasma membrane calcium ATPase, as diagnostic targets for in-vitro detecting Alzheimer's disease.

**8.** Use of protein molecules of SEQ ID NO: 1, said protein molecules being translation products of the gene coding for a plasma membrane calcium ATPase, as screening targets in-vitro for reagents or compounds preventing, or treating, or ameliorating Alzheimer's disease.

**9.** Use of an antibody specifically immunoreactive with a protein molecule of the plasma membrane calcium ATPase having SEQ ID NO: 1, for detecting the pathological state of a cell in a sample obtained from a subject, comprising immunocytochemical staining of said cell with said antibody, wherein an altered degree of staining, or an altered staining pattern in said cell compared to a cell representing a known health status indicates a pathological state of said cell which relates to Alzheimer's disease.

## Patentansprüche

1. Verfahren zum Diagnostizieren oder Prognostizieren der Alzheimer-Krankheit bei einem Patienten oder zum Bestimmen, ob ein Patient ein erhöhtes Risiko hat, diese Krankheit zu entwickeln, oder zum Überwachen der Wirkung einer Behandlung, die einem Patienten mit dieser Krankheit zuteil wurde, umfassend das Bestimmen einer Konzentration und/oder einer Aktivität von

    (i) einem Transcriptionsprodukt des Gens, das für eine Plasmamembran-Calcium-ATPase mit der SEQ ID Nr. 1 codiert; und/oder
    (ii) einem Translationsprodukt des Gens, das für eine PlasmamembranCalcium-ATPase mit der SEQ ID Nr. 1 codiert;

    in einer Probe, die von dem Patienten erhalten wurde, und Vergleichen der Konzentration und/oder der Aktivität mit einem Referenzwert, der einen bekannten Krankheitszustand darstellt, und/oder mit einem Referenzwert, der einen bekannten Gesundheitszustand darstellt, wobei die Konzentration und/oder einer Aktivität von einem Referenzwert, der einen bekannten Gesundheitszustand darstellt, abweicht und/oder einem Referenzwert, der einen bekannten Krankheitszustand darstellt, ähnlich oder gleich ist, wodurch Alzheimer-Krankheit bei dem Patienten diagnostiziert oder prognostiziert wird oder bestimmt wird, ob der Patient ein erhöhtes Risiko hat, diese Krankheit zu entwickeln.

2. Verwendung eines Kits bei einem Verfahren gemäß Anspruch 1 zum Diagnostizieren oder Prognostizieren der Alzheimer-Krankheit oder zum Bestimmen der Prädisposition eines Patienten, eine solche Krankheit zu entwickeln, oder zum Überwachen der Wirkung einer Behandlung, die einem Patienten mit Alzheimer-Krankheit zuteil wurde, wobei der Kit wenigstens ein Reagens umfasst, das aus der Gruppe ausgewählt ist, die aus (i) Reagentien, die ein Transcriptionsprodukt eines Gens, das für eine Plasmamembran-Calcium-ATPase mit der SEQ ID Nr. 1 codiert, nachweisen, und/oder Reagentien, die ein Translationsprodukt des Gens, das für eine Plasmamembran-Calcium-ATPase mit der SEQ ID Nr. 1 codiert, nachweisen, besteht.

3. Screening-Verfahren zum Identifizieren von Mitteln, Modulatoren oder selektiven Antagonisten oder Agonisten zur Verwendung bei der Behandlung von Alzheimer-Krankheit oder verwandter Krankheiten, wobei die Mittel, Modulatoren oder selektiven Antagonisten oder Agonisten die Fähigkeit haben, die Expression oder Konzentration oder Aktivität von einer oder mehreren Substanzen zu verändern, die aus der Gruppe ausgewählt sind, die aus Folgenden besteht:

    (i) einem Gen, das für eine Plasmamembran-Calcium-ATPase mit der SEQ ID Nr. 1 codiert; und/oder
    (ii) einem Transcriptionsprodukt des Gens, das für eine Plasmamembran-Calcium-ATPase mit der SEQ ID Nr. 1 codiert; und/oder
    (iii) einem Translationsprodukt des Gens, das für eine Plasmamembran-Calcium-ATPase mit der SEQ ID Nr. 1 codiert;

    wobei das Verfahren Folgendes umfasst:

    a) In-Kontakt-Bringen einer Zelle mit einer Testverbindung;
    b) Messen der Aktivität und/oder der Konzentration und/oder der Expression von einer oder mehreren Substanzen, die in (i) bis (iii) genannt sind;
    c) Messen der Aktivität und/oder der Konzentration und/oder der Expression von einer oder mehreren Substanzen, die in (i) bis (iii) genannt sind, in einer Kontrollzelle, die nicht mit der Testverbindung in Kontakt gebracht wurde; und Vergleichen der Konzentrationen und/oder Aktivitäten und/oder der Expression der Substanzen in den Zellen der Schritte (b) und (c), wobei eine Veränderung der Aktivität und/oder der Konzentration und/oder der Expression der Substanzen in den kontaktierten Zellen anzeigt, dass die Testverbindung ein Mittel, Modulator oder selektiver Antagonist oder Agonist zur Verwendung bei der Behandlung von Alzheimer-Krankheit oder verwandter Krankheiten ist.

4. Screening-Verfahren zum Identifizieren von Mitteln, Modulatoren oder selektiven Antagonisten oder Agonisten zur Verwendung bei der Behandlung von Alzheimer-Krankheit oder verwandter Krankheiten, wobei die Mittel, Modulatoren oder selektiven Antagonisten oder Agonisten die Fähigkeit haben, die Expression oder Konzentration oder Aktivität von einer oder mehreren Substanzen zu verändern, die aus der Gruppe ausgewählt sind, die aus Folgenden besteht:

(i) einem Gen, das für eine Plasmamembran-Calcium-ATPase mit der SEQ ID Nr. 1 codiert; und/oder
(ii) einem Transcriptionsprodukt des Gens, das für eine Plasmamembran-Calcium-ATPase mit der SEQ ID Nr. 1 codiert; und/oder
(iii) einem Translationsprodukt des Gens, das für eine Plasmamembran-Calcium-ATPase mit der SEQ ID Nr. 1 codiert;

wobei das Verfahren Folgendes umfasst:

a) Verabreichen eines Testverbindung an ein nichthumanes Versuchstier, das prädisponiert ist, Zeichen und Symptome einer neurodegenerativen Krankheit oder verwandter Krankheiten oder Störungen zu entwickeln, oder bereits welche entwickelt hat;
b) Messen der Aktivität und/oder der Konzentration und/oder der Expression von einer oder mehreren Substanzen, die in (i) bis (iii) genannt sind;
c) Messen der Aktivität und/oder der Konzentration und/oder der Expression von einer oder mehreren Substanzen, die in (i) bis (iii) genannt sind, bei einem nichthumanen Kontrolltier, das prädisponiert ist, Zeichen und Symptome einer neurodegenerativen Krankheit oder verwandter Krankheiten oder Störungen zu entwickeln, oder bereits welche entwikkelt hat, wobei diesem nichthumanen Tier keine solche Testverbindung verabreicht wurde;
(d) Vergleichen der Aktivität und/oder der Konzentration und/oder der Expression der Substanzen in den Tieren der Schritte (b) und (c), wobei eine Veränderung der Aktivität und/oder der Konzentration und/oder der Expression der Substanzen in dem nichthumanen Versuchstier anzeigt, dass die Testverbindung ein Mittel, Modulator oder selektiver Antagonist oder Agonist zur Verwendung bei der Behandlung von Alzheimer-Krankheit oder verwandter Krankheiten ist.

**5.** Verfahren gemäß Anspruch 4, wobei das nichthumane Versuchstier und/ oder das nichthumane Kontrolltier ein rekombinantes Tier ist, das eine Plasmamembran- Calcium- ATPase mit der SEQ ID Nr. 1 unter der Kontrolle einer Transcriptionssteuerungselements, bei dem es sich nicht um das Transcriptionssteuerungselement des nativen Plasmamembran- Calcium- ATPase- Gens handelt, exprimiert.

**6.** Verfahren zum Testen einer Verbindung, vorzugsweise zum Durchmustern einer Vielzahl von Verbindungen, um die Bindung der Verbindungen an eine Plasmamembran-Calcium-ATPase mit der SEQ ID Nr. 1 zu bestimmen, wobei das Verfahren die folgenden Schritte umfasst:

(i) Hinzufügen einer flüssigen Suspension der Plasmamembran-Calcium-ATPase oder eines Fragments oder Derivats oder einer Variante davon in eine Vielzahl von Behältern;
(ii) Hinzufügen einer nachweisbaren, insbesondere fluoreszenzmarkierten, Verbindung oder einer Vielzahl von nachweisbaren, insbesondere fluoreszenzmarkierten, Verbindungen, die auf die Bindung hin durchmustert werden sollen, in die Vielzahl von Behältern;
(iii) Inkubieren der Plasmamembran-Calcium-ATPase und der nachweisbaren, insbesondere fluoreszenzmarkierten, Verbindung oder der nachweisbaren, insbesondere fluoreszenzmarkierten, Verbindungen;
(iv) Messen der Mengen vorzugsweise der Fluoreszenz, die mit der Plasmamembran-Calcium-ATPase verbunden ist; und
(v) Bestimmen der Bindung von einer oder mehreren der Verbindungen an die Plasmamembran-Calcium-ATPase.

**7.** Verwendung von Proteinmolekülen der SEQ ID Nr. 1, wobei die Proteinmoleküle Translationsprodukte des Gens, das für eine Plasmamembran-Calcium-ATPase codiert, oder diagnostische Targets für den in-vitro-Nachweis der Alzheimer-Krankheit sind.

**8.** Verwendung von Proteinmolekülen der SEQ ID Nr. 1, wobei die Proteinmoleküle Translationsprodukte des Gens, das für eine Plasmamembran- Calcium- ATPase codiert, sind, als in- vitro- Screening- Targets für Reagentien oder als Verbindungen zur Prävention oder Behandlung oder Linderung der Alzheimer- Krankheit.

**9.** Verwendung eines Antikörpers, der gegenüber einem Proteinmolekül der Plasmamembran-Calcium-ATPase mit der SEQ ID Nr. 1 spezifisch immunreaktiv ist, zum Nachweisen des pathologischen Zustands einer Zelle in einer Probe, die von einem Patienten erhalten wurde, das das immuncytochemische Anfärben der Zelle mit dem Antikörper umfasst, wobei ein veränderter Grad der Anfärbung oder ein verändertes Anfärbungsmuster in der Zelle im Vergleich zu einer Zelle, die einen bekannten Gesundheitszustand repräsentiert, einen pathologischen Zustand der Zelle, der sich auf die Alzheimer-Krankheit bezieht, anzeigt.

**Revendications**

1. Procédé de diagnostic ou de pronostic de la maladie d'Alzheimer chez un sujet, ou de détermination permettant de savoir si un sujet présente un risque accru de développer ladite maladie, ou de contrôle de l'effet d'un traitement administré à un sujet souffrant de ladite maladie, comprenant la détermination d'un taux et/ou d'une activité de

   (i) un produit de transcription du gène codant pour une calcium ATPase de membrane plasmique ayant la SEQ ID NO : 1, et/ou
   (ii) un produit de traduction du gène codant pour une calcium ATPase de membrane plasmique ayant la SEQ ID NO : 1,

   dans un échantillon obtenu à partir dudit sujet et la comparaison dudit taux et/ou de ladite activité à une valeur de référence représentant un état de maladie connu et/ou à une valeur de référence représentant un état de santé connu, et ledit taux et/ou ladite activité varie en comparaison avec une valeur de référence représentant un état de santé connu, et/ou est similaire ou égal à une valeur de référence représentant un état de maladie connu, diagnostiquant ou pronostiquant ainsi la maladie d'Alzheimer chez ledit sujet, ou la détermination permettant de savoir si ledit sujet présente un risque accru de développer la maladie.

2. Utilisation d'un kit dans un procédé selon la revendication 1 permettant de diagnostiquer ou de pronostiquer la maladie d'Alzheimer, ou de déterminer la prédisposition d'un sujet à développer une telle maladie, ou de contrôler l'effet d'un traitement administré à un sujet souffrant de la maladie d'Alzheimer, ledit kit comprenant au moins un réactif qui est choisi dans le groupe consistant en (i) des réactifs qui détectent un produit de transcription d'un gène codant pour une calcium ATPase de membrane plasmique ayant la SEQ ID NO : 1 et/ou (ii) des réactifs qui détectent un produit de traduction du gène codant pour une calcium ATPase de membrane plasmique ayant la SEQ ID NO : 1.

3. Procédé de criblage permettant d'identifier des agents, des modulateurs ou des antagonistes ou agonistes sélectifs pour utilisation dans le traitement de la maladie d'Alzheimer ou de maladies apparentées, lesquels agents, modulateurs ou antagonistes ou agonistes sélectifs ont la capacité de modifier l'expression ou le taux ou l'activité d'une ou plusieurs substances choisies dans le groupe consistant en

   (i) un gène codant pour une calcium ATPase de membrane plasmique ayant la SEQ ID NO : 1, et/ou
   (ii) un produit de transcription du gène codant pour une calcium ATPase de membrane plasmique ayant la SEQ ID NO : 1 et/ou
   (iii) un produit de traduction de gène codant pour une calcium ATPase de membrane plasmique ayant la SEQ ID NO : 1,

   ledit procédé comprend :

   (a) la mise en contact d'une cellule avec un composé d'essai ;
   (b) la mesure de l'activité et/ou du taux et/ou de l'expression d'une ou plusieurs substances citées dans (i) à (iii) ;
   (c) la mesure de l'activité et/ou du taux et/ou de l'expression d'une ou plusieurs substances citées dans (i) à (iii) dans une cellule témoin non mise en contact avec ledit composé d'essai ; et la comparaison des taux et/ou des activités et/ou de l'expression des substances dans les cellules des étapes (b) et (c), où une modification de l'activité et/ou du taux et/ou de l'expression des substances dans les cellules mises en contact indique que le composé d'essai est un agent, un modulateur ou un antagoniste ou agoniste sélectif pour utilisation dans le traitement de la maladie d'Alzheimer ou de maladies apparentées.

4. Procédé de criblage permettant d'identifier des agents, des modulateurs ou des antagonistes ou agonistes sélectifs destinés pour utilisation dans le traitement de la maladie d'Alzheimer ou de maladies apparentées, lesquels agents, modulateurs ou antagonistes ou agonistes sélectifs ont la capacité de modifier l'expression ou le taux ou l'activité d'une ou plusieurs substances choisies dans le groupe consistant en

(i) un gène codant pour une calcium ATPase de membrane plasmique ayant la SEQ ID NO : 1, et/ou
(ii) un produit de transcription du gène codant pour une calcium ATPase de membrane plasmique ayant la SEQ ID NO : 1, et/ou
(iii) un produit de traduction de gène codant pour une calcium ATPase de membrane plasmique ayant la SEQ ID NO : 1,

ledit procédé comprend :

(a) l'administration d'un composé d'essai à un animal expérimental non humain qui est prédisposé à développer ou qui a déjà développé des signes et des symptômes d'une maladie neurodégénérative ou de maladies ou troubles apparentés ;
(b) la mesure de l'activité et/ou du taux et/ou de l'expression d'une ou plusieurs substances citées dans (i) à (iii) ;
(c) la mesure de l'activité et/ou du taux et/ou de l'expression d'une ou plusieurs substances citées dans (i) ou (iii) chez un animal témoin non humain qui est prédisposé à développer ou qui a déjà développé des signes et des symptômes d'une maladie neurodégénérative ou de maladies ou troubles apparentés et auquel animal non humain aucun des composés d'essai n'a été administré ;
(d) la comparaison de l'activité et/ou du taux et/ou de l'expression des substances chez les animaux des étapes (b) et (c), où une modification de l'activité et/ou du taux et/ou de l'expression de substances chez l'animal expérimental non humain indique que le composé d'essai est un agent, un modulateur ou un antagoniste ou agoniste sélectif pour utilisation dans le traitement de la maladie d'Alzheimer ou de maladies apparentées.

**5.** Procédé selon la revendication 4, dans lequel ledit animal expérimental non humain et/ou ledit animal témoin non humain est un animal recombinant qui exprime une calcium ATPase de membrane plasmique ayant la SEQ ID NO : 1, sous le contrôle d'un élément de contrôle transcriptionnel qui n'est pas l'élément de contrôle transcriptionnel du gène de la calcium ATPase de membrane plasmique native.

**6.** Procédé d'essai d'un composé, de préférence de criblage d'une pluralité de composés afin de déterminer la liaison desdits composés à une calcium ATPase de membrane plasmique ayant la SEQ ID NO : 1, ledit procédé comprenant les étapes de :

(i) ajout d'une suspension liquide de ladite calcium ATPase de membrane plasmique, ou d'un fragment, ou d'un dérivé, ou d'un variant de celle-ci, à une pluralité de contenants ;
(ii) ajout d'un composé détectable, en particulier marqué par fluorescence ou d'une pluralité de composés détectables, en particulier marqués par fluorescence à cribler pour ladite liaison à ladite pluralité de contenants ;
(iii) incubation de ladite calcium ATPase de membrane plasmique, et dudit composé détectable, en particulier marqué par fluorescence ou desdits composés détectables, en particulier des composés marqués par fluorescence ;
(iv) mesure de quantités de préférence de la fluorescence associée à ladite calcium ATPase de membrane plasmique ; et
(v) détermination de la liaison par un ou plusieurs desdits composés à ladite calcium ATPase de membrane plasmique.

**7.** Utilisation de molécules protéiques de SEQ ID NO : 1, lesdites molécules protéiques étant des produits de traduction du gène codant pour une calcium ATPase de membrane plasmique, comme cibles diagnostiques permettant de détecter *in vitro* la maladie d'Alzheimer.

**8.** Utilisation de molécules protéiques de SEQ ID NO : 1, lesdites molécules protéiques étant des produits de traduction du gène codant pour une calcium ATPase de membrane plasmique, comme cibles de criblage *in vitro* de réactifs ou de composés empêchant, ou traitant, ou améliorant la maladie d'Alzheimer.

**9.** Utilisation d'un anticorps spécifiquement immunoréactif avec une molécule protéique de la calcium ATPase de membrane plasmique ayant la SEQ ID NO : 1, permettant de détecter l'état pathologique d'une cellule dans un échantillon obtenu à partir d'un sujet, comprenant la coloration immunocytochimique de ladite cellule avec ledit anticorps, où un degré modifié de coloration, ou un motif de coloration modifié dans ladite cellule comparée à une cellule représentant un état de santé connu indique un état pathologique de ladite cellule qui s'apparente à la maladie d'Alzheimer.

**Fig. 1: Identification of differentially expressed genes in a fluorescence differential display screen**

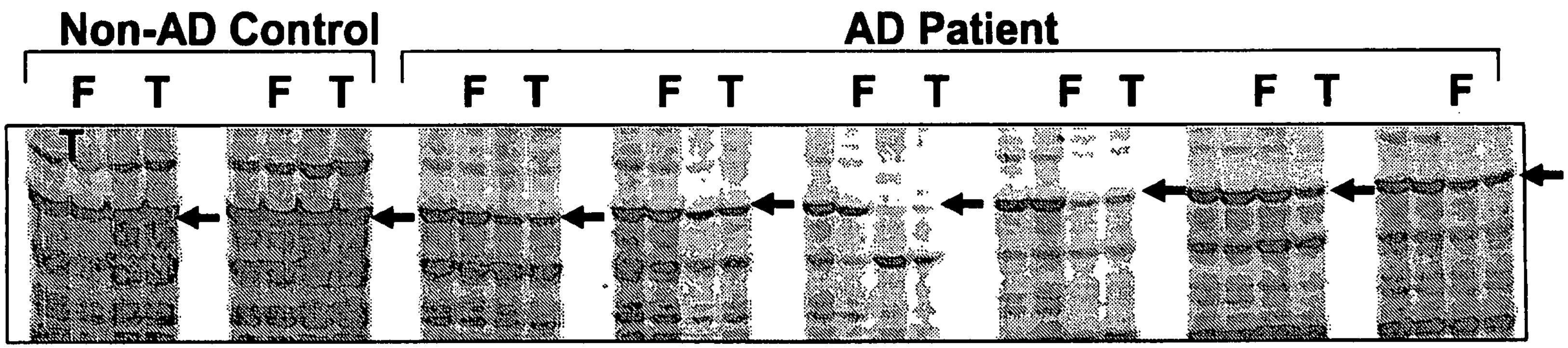

**Fig. 2: Verification of differential expression of ATP2B1 by quantitative PCR**

a)

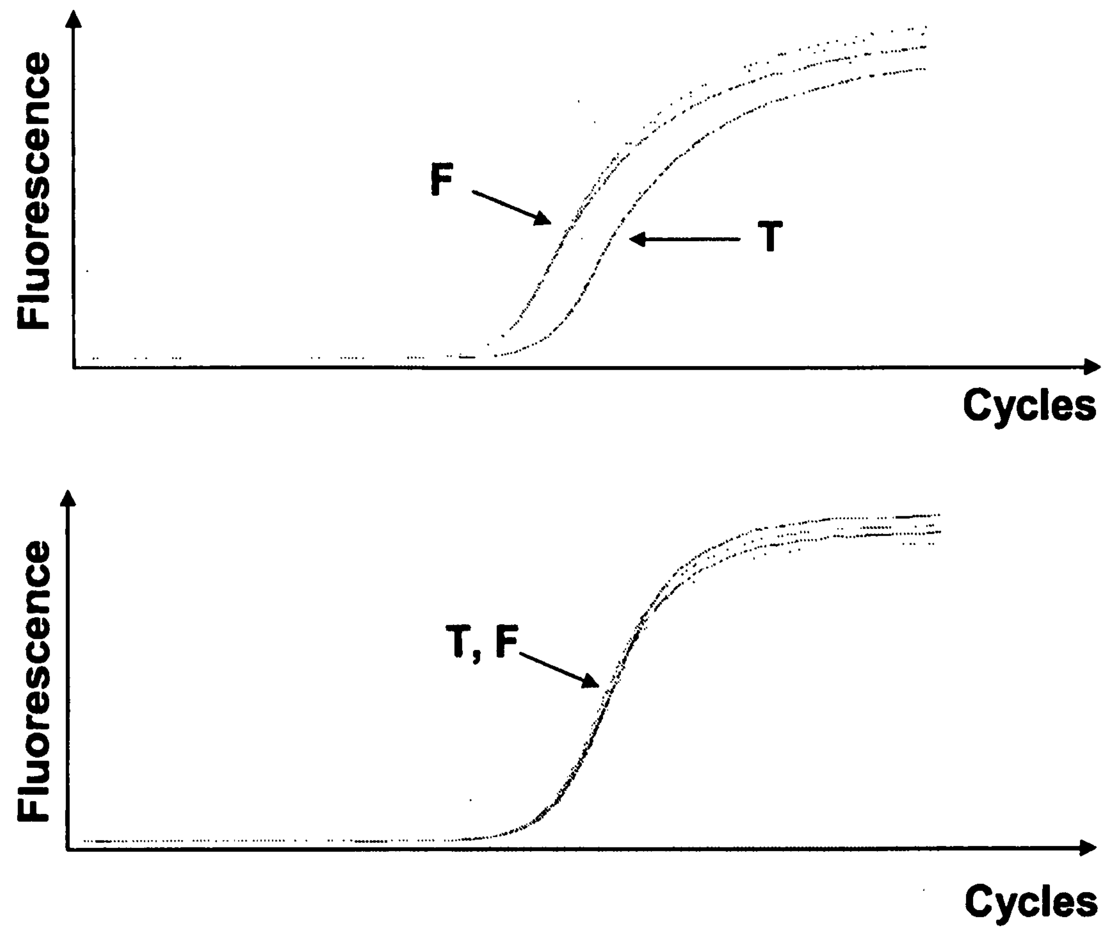

**Fig. 3: Verification of differential expression of ATP2B1 by quantitative PCR**

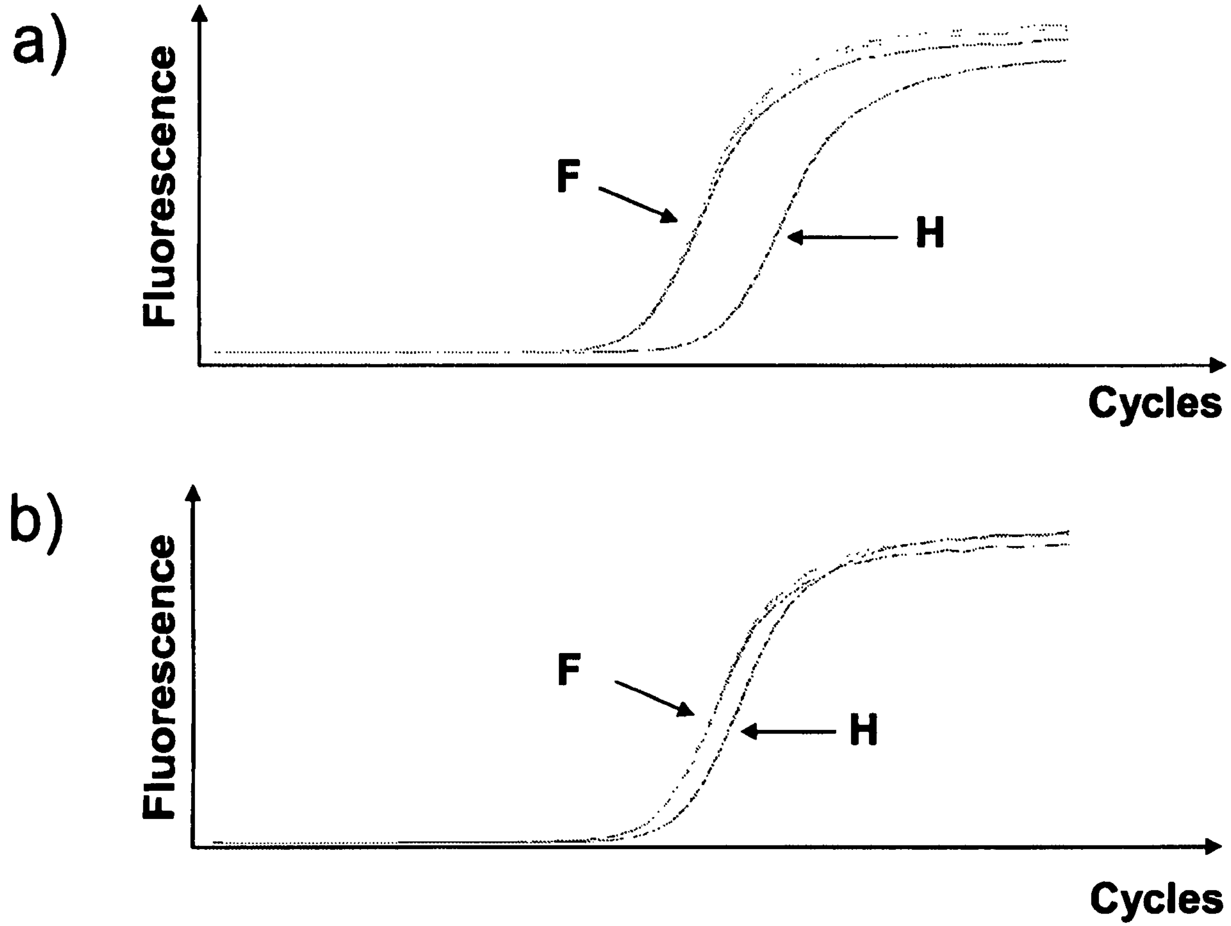

**Fig. 4: Analysis of absolute mRNA expression of ATP2B1**

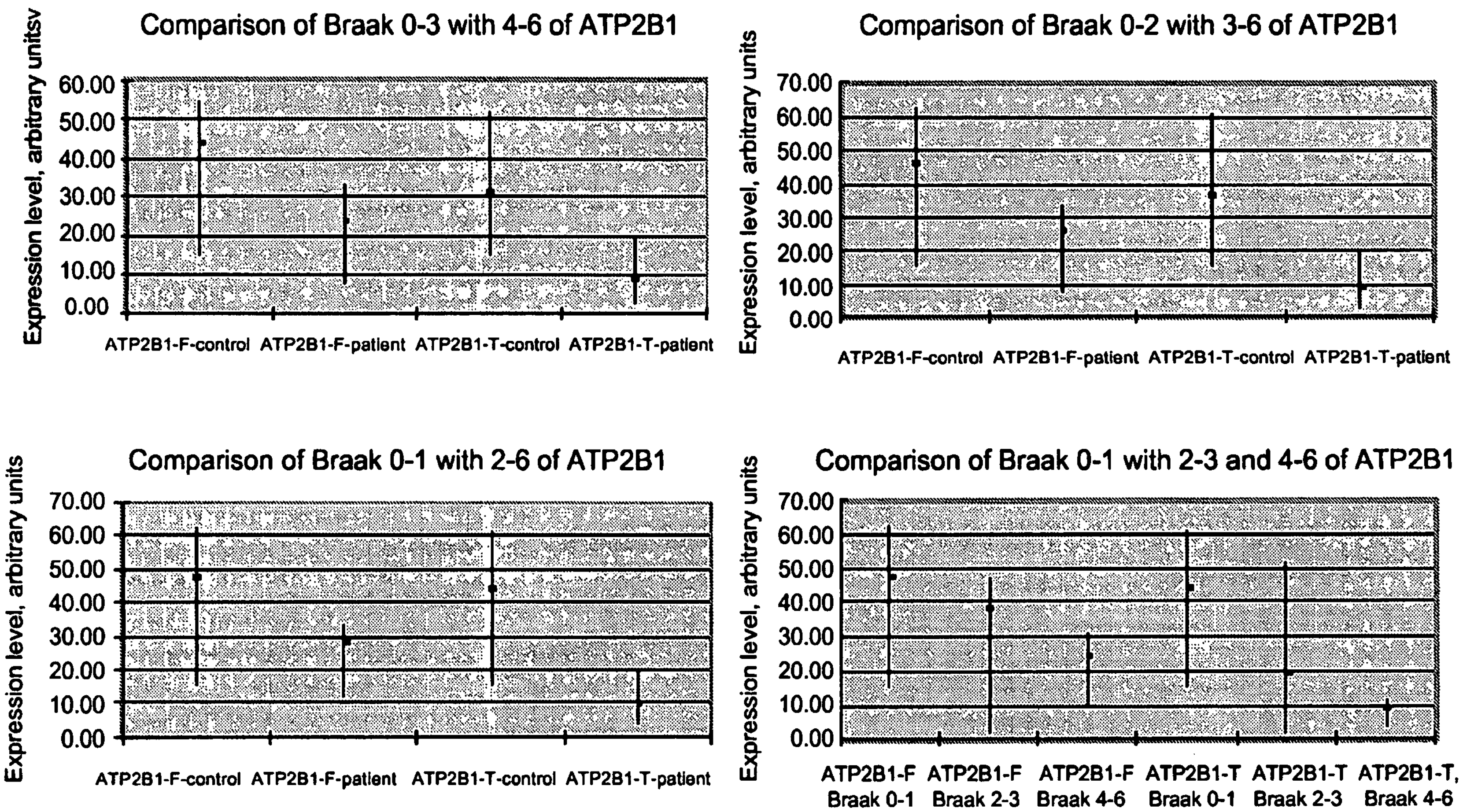

# Fig. 5: SEQ ID NO: 1; amino acid sequence of human ATP2B1 protein

**Length: 1176 aa**

```
   1 MGDMANNSVA YSGVKNSLKE ANHDGDFGIT LAELRALMEL RSTDALRKIQ
  51 ESYGDVYGIC TKLKTSPNEG LSGNPADLER REAVFGKNFI PPKKPKTFLQ
 101 LVWEALQDVT LIILEIAAIV SLGLSFYQPP EGDNALCGEV SVGEEEGEGE
 151 TGWIEGAAIL LSVVCVVLVT AFNDWSKEKQ FRGLQSRIEQ EQKFTVIRGG
 201 QVIQIPVADI TVGDIAQVKY GDLLPADGIL IQGNDLKIDE SSLTGESDHV
 251 KKSLDKDPLL LSGTHVMEGS GRMVVTAVGV NSQTGIIFTL LGAGGEEEEK
 301 KDEKKKEKKN KKQDGAIENR NKAKAQDGAA MEMQPLKSEE GGDGDEKDKK
 351 KANLPKKEKS VLQGKLTKLA VQIGKAGLLM SAITVIILVL YFVIDTFWVQ
 401 KRPWLAECTP IYIQYFVKFF IIGVTVLVVA VPEGLPLAVT ISLAYSVKKM
 451 MKDNNLVRHL DACETMGNAT AICSDKTGTL TMNRMTVVQA YINEKHYKKV
 501 PEPEAIPPNI LSYLVTGISV NCAYTSKILP PEKEGGLPRH VGNKTECALL
 551 GLLLDLKRDY QDVRNEIPEE ALYKVYTFNS VRKSMSTVLK NSDGSYRIFS
 601 KGASEIILKK CFKILSANGE AKVFRPRDRD DIVKTVIEPM ASEGLRTICL
 651 AFRDFPAGEP EPEWDNENDI VTGLTCIAVV GIEDPVRPEV PDAIKKCQRA
 701 GITVRMVTGD NINTARAIAT KCGILHPGED FLCLEGKDFN RRIRNEKGEI
 751 EQERIDKIWP KLRVLARSSP TDKHTLVKGI IDSTVSDQRQ VVAVTGDGTN
 801 DGPALKKADV GFAMGIAGTD VAKEASDIIL TDDNFTSIVK AVMWGRNVYD
 851 SISKFLQFQL TVNVVAVIVA FTGACITQDS PLKAVQMLWV NLIMDTLASL
 901 ALATEPPTES LLLRKPYGRN KPLISRTMMK NILGHAFYQL VVVFTLLFAG
 951 EKFFDIDSGR NAPLHAPPSE HYTIVFNTFV LMQLFNEINA RKIHGERNVF
1001 EGIFNNAIFC TIVLGTFVVQ IIIVQFGGKP FSCSELSIEQ WLWSIFLGMG
1051 TLLWGQLIST IPTSRLKFLK EAGHGTQKEE IPEEELAEDV EEIDHAEREL
1101 RRGQILWFRG LNRIQTQMDV VNAFQSGSSI QGALRRQPSI ASQHHDVTNI
1151 STPTHVVFSS STASTTVGYS SGECIS
```

# Fig. 6: SEQ ID NO: 2, nucleotide sequence of human ATP2B1 cDNA

**Length: 5178 bp**

```
   1 GGCCAAAGGT CAAGATACTT CTCTGGGAAA TGTTGCTGCT GATGCTGCTT
  51 TACAAAGTCA TACAATGAGT GTTTGGTTTA AGAAAGATTT TCATACTTAA
 101 AAGATTTTCA TCTTGGAAAT ACATCAAGTG AAAATTAAAT TCTTTTGGGA
 151 AACATTTTCC TTCTGATATA TTATACTTGT AATGGGCGAC ATGGCAAACA
 201 ACTCAGTTGC TTACAGTGGT GTGAAAAACT CTTTGAAGGA AGCTAATCAT
 251 GATGGAGACT TTGGAATTAC GCTCGCAGAG CTGCGGGCTC TCATGGAGCT
 301 CAGGTCCACA GATGCATTAC GAAAAATACA GGAAAGCTAT GGAGATGTCT
 351 ATGGAATTTG CACCAAATTG AAAACATCTC CCAATGAAGG TTTAAGTGGA
 401 AACCCTGCAG ATTTAGAAAG AAGAGAAGCA GTGTTTGGAA AGAATTTTAT
 451 ACCTCCTAAA AAGCCAAAAA CCTTTCTTCA ATTAGTATGG GAAGCATTAC
 501 AAGATGTCAC TTTAATTATA TTAGAAATTG CAGCCATAGT ATCATTGGGC
 551 CTTTCTTTTT ATCAGCCTCC AGAAGGGGAT AATGCACTTT GTGGAGAAGT
 601 TTCTGTTGGG GAGGAAGAAG GTGAAGGTGA AACTGGTTGG ATTGAAGGAG
 651 CTGCAATCCT CTTGTCTGTA GTGTGTGTGG TGTTAGTAAC AGCTTTCAAT
 701 GACTGGAGTA AGGAAAAACA GTTTAGAGGT TTGCAGAGCC GAATTGAACA
 751 AGAACAGAAG TTCACTGTCA TCAGGGGTGG TCAGGTCATT CAGATACCTG
 801 TAGCTGACAT TACTGTTGGA GATATTGCTC AAGTGAAATA TGGTGATCTT
 851 CTTCCAGCTG ACGGCATACT TATTCAAGGC AACGATCTTA AAATTGATGA
 901 AAGCTCATTG ACTGGTGAAT CAGATCATGT TAAAAAGTCT TTAGATAAGG
 951 ATCCCTTACT TCTATCAGGT ACTCATGTAA TGGAAGGCTC TGGAAGAATG
1001 GTAGTTACAG CTGTAGGTGT AAATTCTCAA ACTGGAATTA TCTTTACCTT
1051 ACTTGGAGCT GGAGGTGAAG AGGAAGAGAA GAAAGATGAG AAGAAAAAGG
1101 AAAAGAAAAA TAAGAAACAA GATGGAGCTA TTGAGAATCG CAACAAAGCA
1151 AAAGCCCAGG ATGGTGCAGC CATGGAAATG CAGCCATTGA AGAGTGAAGA
1201 AGGTGGAGAT GGTGATGAAA AAGATAAAAA GAAAGCAAAT TTGCCAAAAA
1251 AGGAAAAATC TGTTTTACAA GGGAAACTTA CAAAACTGGC TGTTCAGATT
1301 GGCAAAGCAG GTCTGTTGAT GTCTGCCATC ACAGTTATCA TTCTAGTATT
1351 ATATTTTGTC ATTGACACCT TCTGGGTTCA GAAAAGACCA TGGCTTGCTG
1401 AGTGCACACC AATTTATATA CAATACTTTG TGAAGTTCTT CATTATTGGA
1451 GTTACAGTTT TAGTGGTCGC AGTGCCAGAA GGTCTTCCAC TTGCAGTCAC
1501 GATCTCACTG GCTTATTCAG TCAAAAAAAT GATGAAAGAT AATAACTTAG
1551 TAAGGCATCT GGATGCTTGT GAAACCATGG GAAATGCTAC AGCTATTTGT
1601 TCAGATAAAA CAGGAACTTT GACAATGAAC AGAATGACAG TCGTTCAAGC
1651 TTACATAAAT GAAAAACATT ATAAAAAGGT TCCTGAACCA GAAGCTATTC
1701 CACCAAATAT TTTGTCCTAT CTTGTAACAG GAATTTCTGT GAATTGTGCT
1751 TATACATCAA AAATATTGCC ACCAGAGAAA GAGGGTGGAT TACCTCGTCA
1801 CGTTGGTAAT AAAACTGAAT GTGCCTTGTT GGGACTTCTT TTGGATTTAA
1851 AACGGGATTA TCAGGATGTT AGAAATGAAA TACCAGAAGA AGCACTGTAC
1901 AAAGTCTACA CCTTCAATTC TGTTAGGAAG TCCATGAGTA CTGTCCTGAA
1951 AAATTCAGAT GGAAGTTATC GAATATTCAG CAAGGGTGCA TCTGAGATAA
2001 TTCTGAAAAA GTGTTTCAAA ATCTTGAGTG CTAATGGTGA GGCAAAAGTA
2051 TTCAGACCAA GGGACCGTGA TGATATTGTA AAAACTGTGA TTGAACCGAT
2101 GGCATCAGAA GGCTTGAGAA CCATATGTCT TGCATTCAGA GATTTTCCAG
2151 CAGGAGAACC AGAACCAGAG TGGGATAATG AAAATGATAT TGTCACCGGC
2201 CTTACATGCA TTGCTGTTGT GGGGATTGAA GATCCTGTGA GACCTGAGGT
2251 GCCAGATGCA ATTAAAAAGT GTCAGAGGGC TGGAATTACT GTGCGGATGG
2301 TCACTGGTGA TAATATTAAT ACTGCTCGGG CCATTGCTAC CAAATGTGGT
```

```
2351 ATTTTACATC CTGGGGAAGA TTTTCTGTGC CTAGAAGGTA AAGATTTTAA
2401 CAGAAGAATA CGAAATGAAA AAGGAGAGAT TGAGCAAGAG AGGATAGACA
2451 AGATTTGGCC AAAACTTCGA GTACTTGCAA GATCATCTCC TACTGATAAG
2501 CATACACTGG TTAAAGGTAT AATTGACAGC ACTGTCTCAG ACCAACGCCA
2551 GGTTGTAGCT GTAACTGGTG ATGGTACAAA TGATGGCCCA GCACTAAAGA
2601 AAGCAGATGT TGGATTTGCA ATGGGTATTG CTGGAACTGA TGTAGCTAAA
2651 GAAGCATCCG ATATTATTCT CACAGATGAC AACTTTACAA GCATTGTTAA
2701 AGCAGTTATG TGGGGACGAA ATGTCTATGA CAGCATCTCA AAATTCCTTC
2751 AGTTCCAACT TACTGTTAAT GTAGTAGCAG TGATTGTTGC TTTTACGGGC
2801 GCCTGCATTA CTCAAGACTC ACCGCTTAAG GCTGTGCAGA TGCTGTGGGT
2851 AAACCTCATA ATGGATACAC TCGCTTCCCT GGCTCTGGCA ACGGAACCAC
2901 CCACTGAGTC TCTCTTGCTT CGGAAACCTT ATGGTAGAAA TAAGCCTCTC
2951 ATCTCACGTA CAATGATGAA GAATATTTTG GGTCATGCAT TCTATCAACT
3001 TGTAGTAGTC TTTACACTCT TATTTGCTGG AGAAAAGTTT TTTGACATTG
3051 ATAGTGGAAG AAATGCTCCT TTGCATGCTC CTCCTTCAGA ACATTATACT
3101 ATTGTTTTTA ATACCTTTGT GCTGATGCAA CTTTTCAACG AAATAAATGC
3151 CCGGAAAATT CATGGTGAAA GAAATGTATT CGAAGGAATC TTTAACAATG
3201 CCATCTTCTG CACAATTGTT TTAGGCACTT TTGTGGTACA GATAATAATT
3251 GTGCAGTTTG GTGGAAAACC TTTCAGTTGT TCAGAACTTT CAATAGAACA
3301 GTGGCTATGG TCAATATTCC TAGGAATGGG AACATTACTC TGGGGCCAGC
3351 TTATTTCAAC AATTCCAACT AGCCGTTTAA AATTCCTCAA AGAAGCTGGT
3401 CATGGAACAC AAAAGGAAGA AATACCTGAG GAGGAATTAG CAGAGGATGT
3451 TGAAGAGATT GATCACGCTG AAAGGGAGTT GCGGCGTGGC CAAATCTTGT
3501 GGTTTAGAGG TCTGAACAGA ATCCAAACAC AGATGGATGT AGTGAATGCT
3551 TTCCAGAGTG GAAGTTCCAT TCAGGGGGCT CTAAGGCGGC AACCCTCCAT
3601 CGCCAGCCAG CATCATGATG TAACAAATAT TTCTACCCCT ACACATGTAG
3651 TGTTTTCCTC TTCTACTGCT TCTACTACTG TGGGGTATTC GAGTGGTGAA
3701 TGCATTTCGT AGTTCTTTAT ATGAAGGGTT AGAAAAACCG GAATCAAGAA
3751 GTTCGATTCA CAACTTTATG ACACATCCTG AGTTTAGGAT AGAAGATTCA
3801 GAGCCTCATA TCCCCCTTAT TGATGACACT GATGCCGAAG ATGATGCTCC
3851 TACAAAACGT AACTCCAGTC CTCCACCCTC TCCCAACAAA AATAACAATG
3901 CTGTTGACAG TGGAATTCAC CTTACAATAG AAATGAACAA GTCTGCTACC
3951 TCTTCATCCC CAGGAAGCCC ACTACATAGT TTGGAAACAT CACTCTGATT
4001 GTAAGCTGAA TGTTAACACA CTAGCTGCAT TGTAAAGAAA CAAATTGAAA
4051 CTGGGTCTTT TCACATATTG TGATGGACAA GCTAGTATTC TTGTCTTTGG
4101 ACTTCAACAG AAGACACACT TGTACGAATG TAGATTTATT TTTTTAAAAA
4151 AAAAAAAGCA AAGCTTTCTG CCAGACTGAG GGTGCTTTTT GGGGGGTGGG
4201 AGAAATGAAC TGACAGATAA ACAGTAACTC AGCGTAAGTG ACCTGTGGAT
4251 CATCAGTAGT ACCCAAGCAT GGTCCTGAAC AGTGTATTAG CAGAGCTTTA
4301 GTTTATCTCA GTCCTGGATG GGAGAGAGAG GGAGGAGTAA AACTGGGCAA
4351 GACAAAGAGC CCTGGATCGT TGAATTGATA GTTTAATTTC TGCTGTTGGC
4401 TGTTAATAAT TTGGATTATT TATGTTTATA AATGATACAG ATCTGTTTAC
4451 AAGGTTTGTA GATACTTTTT TTGTTCCTGT TCATAGATGG GAAGCTTCCT
4501 TATAACTGAT GCAGAGAAAA ATTAGTCCTT CAAATACTGC TGTATTTTCA
4551 GGAAAAAAAA AAAAAGGGGT GTTTCTATTG AAACTGTACT AAATTTTTGC
4601 CTACAATTTA CCTTGTTAAA TATTGTAGAT AAATTGCTAA TACTAATAGC
4651 CAAATAGATA ACACTAATGC TTTGTTTAAA AAAACGAAAA AAACAAAACA
4701 AAACAAAAAA ACATGAGCAG TGGTGTTCTA ATGAAGTTAT GACATCTGTC
4751 CTAATTAGTT TCTTAAATAC ATTTACTACT TAATGGTTTT GAAGCAACTG
4801 TTTAATTTTT AAAATTTTTG AAAACTTGCT AATAACCTTT TGTTCAGAAT
4851 TTAGTAGATT GTTTAATGCA GGACATCAAC TACATAATGA AAGATGCTTG
4901 AAATGCTTTT GTTATTTCAG GCAAATCAAA TTAAATCAGA CTATCAAACT
4951 ACAAGAGAAC CTTAGTCTTT TTTGTTTTTG TCTAAACATG TTAGTTTAGT
5001 GATGTCTTGG TGAACTGTGA GTGAACTGTA TTGATTTTTC TAATCTTTAG
```

5051 AGACCTTCAC ACAGCATGAG GGCTGTTGGC ATTTTGAAAA AGGTTAATAA
5101 GTAGAAGCAT ACATGTTTTC CTTTTGTTTT TGAAACTTGT TTGTAAACAT
5151 AAATAAATAT GCCCTTTTAT TAAATAAA

# Fig. 7: SEQ ID NO: 3

**Length: 644 bp**

```
  1  GGAGTTTAGA GGTCTGAACA GAATCCAAAC ACAGATGGAT GTAGTGAATG
 51  CTTTCCAGAG TGGAAGTTCC ATTCAGGGGG CTCTAAGGCG GCAACCCTCC
101  ATCGCCAGCC AGCATCATGA TGTAACAAAT ATTTCTACCC CTACACATGT
151  AGTGTTTTCC TCTTCTACTG CTTCTACTAC TGTGGGATTC GAGTGGTGAA
201  TGCATTTCGT AGTTCTTTAT ATGAAGGGTT AGAAAAACCG GAATCAAGAA
251  GTTCGATTCA CAACTTTATG ACACATCCTG AGTTTAGGAT AGAAGATTCA
301  GAGCCTCATA TCCCCCTTAT TGATGACACT GATGCCGAAG ATGATGCTCC
351  TACAAAACGT AACTCCAGTC CTCCGCCCTC TCCCAACAAA AATAACAATG
401  CTGTTGACAG TGGAATTCAC CTTACAATAG AAATGAACAA GTCTGCTACC
451  TCTTCATCCC CAGGAAGCCC ACTACATAGT TTGGAAACAT CACTCTGATT
501  GTAAGCTGAA TGTTAACACA CTAGCTGCAT TGTAAAGAAA CAAATTGAAA
551  CTGGGTCTTT TCACATATTG TGATGGACAA GCTAGTATTT TTGGCTTTGG
601  ACTTCAACAG AAGAACACCT TNTCGAATGN AGATTTATTT TTTT
```

# Fig. 8: SEQ ID NO: 4, nucleotide sequence of human ATP2B1 coding sequence

**Length: 3531 bp**

```
   1 ATGGGCGACA TGGCAAACAA CTCAGTTGCT TACAGTGGTG TGAAAAACTC
  51 TTTGAAGGAA GCTAATCATG ATGGAGACTT TGGAATTACG CTCGCAGAGC
 101 TGCGGGCTCT CATGGAGCTC AGGTCCACAG ATGCATTACG AAAAATACAG
 151 GAAAGCTATG GAGATGTCTA TGGAATTTGC ACCAAATTGA AAACATCTCC
 201 CAATGAAGGT TTAAGTGGAA ACCCTGCAGA TTTAGAAAGA AGAGAAGCAG
 251 TGTTTGGAAA GAATTTTATA CCTCCTAAAA AGCCAAAAAC CTTTCTTCAA
 301 TTAGTATGGG AAGCATTACA AGATGTCACT TTAATTATAT TAGAAATTGC
 351 AGCCATAGTA TCATTGGGCC TTTCTTTTTA TCAGCCTCCA GAAGGGGATA
 401 ATGCACTTTG TGGAGAAGTT TCTGTTGGGG AGGAAGAAGG TGAAGGTGAA
 451 ACTGGTTGGA TTGAAGGAGC TGCAATCCTC TTGTCTGTAG TGTGTGTGGT
 501 GTTAGTAACA GCTTTCAATG ACTGGAGTAA GGAAAAACAG TTTAGAGGTT
 551 TGCAGAGCCG AATTGAACAA GAACAGAAGT TCACTGTCAT CAGGGGTGGT
 601 CAGGTCATTC AGATACCTGT AGCTGACATT ACTGTTGGAG ATATTGCTCA
 651 AGTGAAATAT GGTGATCTTC TTCCAGCTGA CGGCATACTT ATTCAAGGCA
 701 ACGATCTTAA AATTGATGAA AGCTCATTGA CTGGTGAATC AGATCATGTT
 751 AAAAAGTCTT TAGATAAGGA TCCCTTACTT CTATCAGGTA CTCATGTAAT
 801 GGAAGGCTCT GGAAGAATGG TAGTTACAGC TGTAGGTGTA AATTCTCAAA
 851 CTGGAATTAT CTTTACCTTA CTTGGAGCTG GAGGTGAAGA GGAAGAGAAG
 901 AAAGATGAGA AGAAAAAGGA AAAGAAAAAT AAGAAACAAG ATGGAGCTAT
 951 TGAGAATCGC AACAAAGCAA AAGCCCAGGA TGGTGCAGCC ATGGAAATGC
1001 AGCCATTGAA GAGTGAAGAA GGTGGAGATG GTGATGAAAA AGATAAAAAG
1051 AAAGCAAATT TGCCAAAAAA GGAAAAATCT GTTTTACAAG GGAAACTTAC
1101 AAAACTGGCT GTTCAGATTG GCAAAGCAGG TCTGTTGATG TCTGCCATCA
1151 CAGTTATCAT TCTAGTATTA TATTTTGTCA TTGACACCTT CTGGGTTCAG
1201 AAAAGACCAT GGCTTGCTGA GTGCACACCA ATTTATATAC AATACTTTGT
1251 GAAGTTCTTC ATTATTGGAG TTACAGTTTT AGTGGTCGCA GTGCCAGAAG
1301 GTCTTCCACT TGCAGTCACG ATCTCACTGG CTTATTCAGT CAAAAAAATG
1351 ATGAAAGATA ATAACTTAGT AAGGCATCTG GATGCTTGTG AAACCATGGG
1401 AAATGCTACA GCTATTTGTT CAGATAAAAC AGGAACTTTG ACAATGAACA
1451 GAATGACAGT CGTTCAAGCT TACATAAATG AAAAACATTA TAAAAAGGTT
1501 CCTGAACCAG AAGCTATTCC ACCAAATATT TTGTCCTATC TTGTAACAGG
1551 AATTTCTGTG AATTGTGCTT ATACATCAAA AATATTGCCA CCAGAGAAAG
1601 AGGGTGGATT ACCTCGTCAC GTTGGTAATA AAACTGAATG TGCCTTGTTG
1651 GGACTTCTTT TGGATTTAAA ACGGGATTAT CAGGATGTTA GAAATGAAAT
1701 ACCAGAAGAA GCACTGTACA AAGTCTACAC CTTCAATTCT GTTAGGAAGT
1751 CCATGAGTAC TGTCCTGAAA AATTCAGATG GAAGTTATCG AATATTCAGC
1801 AAGGGTGCAT CTGAGATAAT TCTGAAAAAG TGTTTCAAAA TCTTGAGTGC
1851 TAATGGTGAG GCAAAAGTAT TCAGACCAAG GGACCGTGAT GATATTGTAA
1901 AAACTGTGAT TGAACCGATG GCATCAGAAG GCTTGAGAAC CATATGTCTT
1951 GCATTCAGAG ATTTTCCAGC AGGAGAACCA GAACCAGAGT GGGATAATGA
2001 AAATGATATT GTCACCGGCC TTACATGCAT TGCTGTTGTG GGGATTGAAG
2051 ATCCTGTGAG ACCTGAGGTG CCAGATGCAA TTAAAAAGTG TCAGAGGGCT
2101 GGAATTACTG TGCGGATGGT CACTGGTGAT AATATTAATA CTGCTCGGGC
2151 CATTGCTACC AAATGTGGTA TTTTACATCC TGGGGAAGAT TTTCTGTGCC
2201 TAGAAGGTAA AGATTTTAAC AGAAGAATAC GAAATGAAAA AGGAGAGATT
2251 GAGCAAGAGA GGATAGACAA GATTTGGCCA AAACTTCGAG TACTTGCAAG
2301 ATCATCTCCT ACTGATAAGC ATACACTGGT TAAAGGTATA ATTGACAGCA
2351 CTGTCTCAGA CCAACGCCAG GTTGTAGCTG TAACTGGTGA TGGTACAAAT
2401 GATGGCCCAG CACTAAAGAA AGCAGATGTT GGATTTGCAA TGGGTATTGC
2451 TGGAACTGAT GTAGCTAAAG AAGCATCCGA TATTATTCTC ACAGATGACA
2501 ACTTTACAAG CATTGTTAAA GCAGTTATGT GGGGACGAAA TGTCTATGAC
```

2551 AGCATCTCAA AATTCCTTCA GTTCCAACTT ACTGTTAATG TAGTAGCAGT
2601 GATTGTTGCT TTTACGGGCG CCTGCATTAC TCAAGACTCA CCGCTTAAGG
2651 CTGTGCAGAT GCTGTGGGTA AACCTCATAA TGGATACACT CGCTTCCCTG
2701 GCTCTGGCAA CGGAACCACC CACTGAGTCT CTCTTGCTTC GGAAACCTTA
2751 TGGTAGAAAT AAGCCTCTCA TCTCACGTAC AATGATGAAG AATATTTTGG
2801 GTCATGCATT CTATCAACTT GTAGTAGTCT TTACACTCTT ATTTGCTGGA
2851 GAAAAGTTTT TTGACATTGA TAGTGGAAGA AATGCTCCTT TGCATGCTCC
2901 TCCTTCAGAA CATTATACTA TTGTTTTTAA TACCTTTGTG CTGATGCAAC
2951 TTTTCAACGA AATAAATGCC CGGAAAATTC ATGGTGAAAG AAATGTATTC
3001 GAAGGAATCT TTAACAATGC CATCTTCTGC ACAATTGTTT TAGGCACTTT
3051 TGTGGTACAG ATAATAATTG TGCAGTTTGG TGGAAAACCT TTCAGTTGTT
3101 CAGAACTTTC AATAGAACAG TGGCTATGGT CAATATTCCT AGGAATGGGA
3151 ACATTACTCT GGGGCCAGCT TATTTCAACA ATTCCAACTA GCCGTTTAAA
3201 ATTCCTCAAA GAAGCTGGTC ATGGAACACA AAAGGAAGAA ATACCTGAGG
3251 AGGAATTAGC AGAGGATGTT GAAGAGATTG ATCACGCTGA AAGGGAGTTG
3301 CGGCGTGGCC AAATCTTGTG GTTTAGAGGT CTGAACAGAA TCCAAACACA
3351 GATGGATGTA GTGAATGCTT TCCAGAGTGG AAGTTCCATT CAGGGGGCTC
3401 TAAGGCGGCA ACCCTCCATC GCCAGCCAGC ATCATGATGT AACAAATATT
3451 TCTACCCCTA CACATGTAGT GTTTTCCTCT TCTACTGCTT CTACTACTGT
3501 GGGGTATTCG AGTGGTGAAT GCATTTCGTA G

# Fig. 9: Alignment of SEQ ID NO: 3, with human ATP2B1 cDNA

**Length: 640 bp**

```
   4 GTTTAGAGGTCTGAACAGAATCCAAACACAGATGGATGTAGTGAATGCTT 53
     ||||||||||||||||||||||||||||||||||||||||||||||||||
3502 GTTTAGAGGTCTGAACAGAATCCAAACACAGATGGATGTAGTGAATGCTT 3551
              .         .         .         .         .
  54 TCCAGAGTGGAAGTTCCATTCAGGGGGCTCTAAGGCGGCAACCCTCCATC 103
     ||||||||||||||||||||||||||||||||||||||||||||||||||
3552 TCCAGAGTGGAAGTTCCATTCAGGGGGCTCTAAGGCGGCAACCCTCCATC 3601
              .         .         .         .         .
 104 GCCAGCCAGCATCATGATGTAACAAATATTTCTACCCCTACACATGTAGT 153
     ||||||||||||||||||||||||||||||||||||||||||||||||||
3602 GCCAGCCAGCATCATGATGTAACAAATATTTCTACCCCTACACATGTAGT 3651
              .         .         .         .         .
 154 GTTTTCCTCTTCTACTGCTTCTACTACTGTGGG..ATTCGAGTGGTGAAT 201
     |||||||||||||||||||||||||||||||||  |||||||||||||||
3652 GTTTTCCTCTTCTACTGCTTCTACTACTGTGGGGTATTCGAGTGGTGAAT 3701
              .         .         .         .         .
 202 GCATTTCGTAGTTCTTTATATGAAGGGTTAGAAAAACCGGAATCAAGAAG 251
     ||||||||||||||||||||||||||||||||||||||||||||||||||
3702 GCATTTCGTAGTTCTTTATATGAAGGGTTAGAAAAACCGGAATCAAGAAG 3751
              .         .         .         .         .
 252 TTCGATTCACAACTTTATGACACATCCTGAGTTTAGGATAGAAGATTCAG 301
     ||||||||||||||||||||||||||||||||||||||||||||||||||
3752 TTCGATTCACAACTTTATGACACATCCTGAGTTTAGGATAGAAGATTCAG 3801
              .         .         .         .         .
 302 AGCCTCATATCCCCCTTATTGATGACACTGATGCCGAAGATGATGCTCCT 351
     ||||||||||||||||||||||||||||||||||||||||||||||||||
3802 AGCCTCATATCCCCCTTATTGATGACACTGATGCCGAAGATGATGCTCCT 3851
              .         .         .         .         .
 352 ACAAAACGTAACTCCAGTCCTCCGCCCTCTCCCAACAAAAATAACAATGC 401
     ||||||||||||||||||||||| ||||||||||||||||||||||||||
3852 ACAAAACGTAACTCCAGTCCTCCACCCTCTCCCAACAAAAATAACAATGC 3901
              .         .         .         .         .
 402 TGTTGACAGTGGAATTCACCTTACAATAGAAATGAACAAGTCTGCTACCT 451
     ||||||||||||||||||||||||||||||||||||||||||||||||||
3902 TGTTGACAGTGGAATTCACCTTACAATAGAAATGAACAAGTCTGCTACCT 3951
              .         .         .         .         .
 452 CTTCATCCCCAGGAAGCCCACTACATAGTTTGGAAACATCACTCTGATTG 501
     ||||||||||||||||||||||||||||||||||||||||||||||||||
3952 CTTCATCCCCAGGAAGCCCACTACATAGTTTGGAAACATCACTCTGATTG 4001
              .         .         .         .         .
 502 TAAGCTGAATGTTAACACACTAGCTGCATTGTAAAGAAACAAATTGAAAC 551
     ||||||||||||||||||||||||||||||||||||||||||||||||||
4002 TAAGCTGAATGTTAACACACTAGCTGCATTGTAAAGAAACAAATTGAAAC 4051
              .         .         .         .         .
 552 TGGGTCTTTTCACATATTGTGATGGACAAGCTAGTATTTTTGGCTTTGGA 601
     |||||||||||||||||||||||||||||||||||||| ||| |||||||
4052 TGGGTCTTTTCACATATTGTGATGGACAAGCTAGTATTCTTGTCTTTGGA 4101
              .         .         .         .
 602 CTTCAACAGAAGA.ACACCTTNCGAATGNAGATTTATTTTTTT 644
     |||||||||||| |||| | : ||||||:||||||||||||||
4102 CTTCAACAGAAGACACACTTGTACGAATGTAGATTTATTTTTTT 4145
```

**Fig. 10: Schematic alignment of SEQ ID NO: 2 and ATP2B1 cds, SEQ ID NO: 4 with Genome Database EST-cluster and mRNAs**

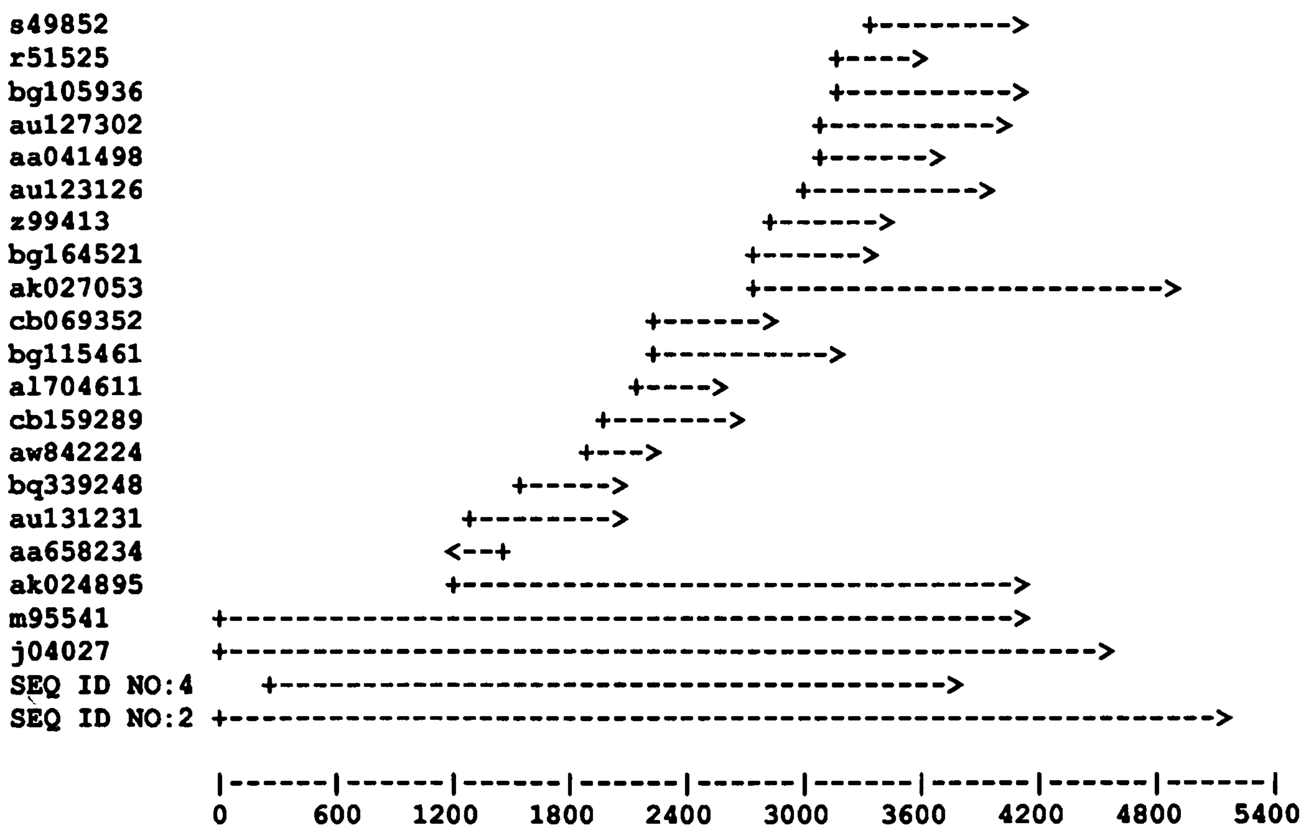

**Fig. 11: Schematic alignment of SEQ ID NO: 2, SEQ ID NO: 3, coding sequence and RT-PCR primer sequences of ATP2B1**

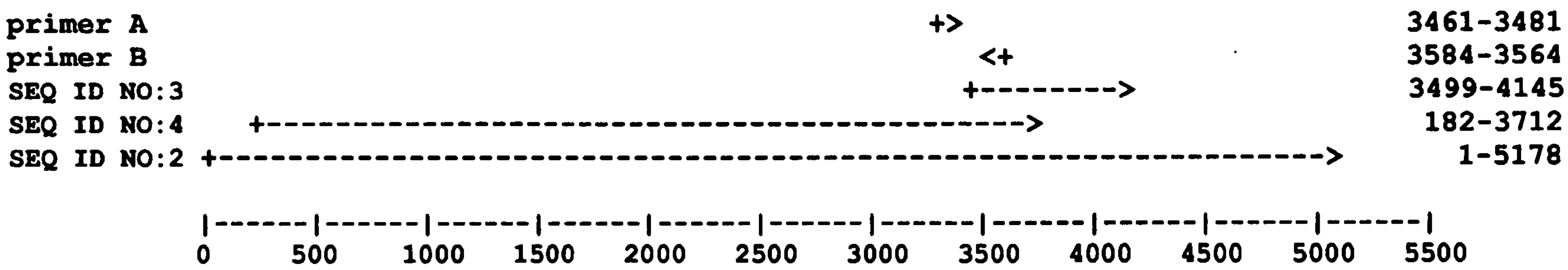


\+ 5'-end of sequence
\> 3'-end of sequence

Fig. 12 :

**Samples** — **Controls** (Braak stages 0–2) / **AD Patients** (Braak stages 3–6)

| Braak stage | 0 | | 1 | | 2 | | 3 | | 4 | | 5 | | 6 | |
|---|---|---|---|---|---|---|---|---|---|---|---|---|---|---|
| | C011 | F1.25 | C005 | F1.08 | C008 | F1.14 | C025 | F1.02 | P012 | F1.43 | P010 | F6.67 | P014 | F2.33 |
| | C012 | T1.09 | C014 | F1.27 | C031 | F1.32 | C035 | F1.56 | P016 | F3.12 | P011 | F2.44 | P017 | F2.00 |
| | C026 | F1.12 | C028 | F1.30 | C033 | T1.77 | DE05 | F2.70 | P038 | F2.08 | P040 | F3.12 | P019 | F2.86 |
| | C027 | T1.12 | C029 | F1.09 | C034 | F1.10 | DE07 | F2.44 | P046 | F3.70 | P041 | F2.56 | P042 | F1.64 |
| | C032 | F1.08 | C030 | F1.01 | C041 | T1.07 | | | P047 | F2.70 | P048 | T1.00 | | |
| | | | C036 | F1.22 | C042 | F1.27 | | | | | P049 | F6.07 | | |
| | | | C038 | F2.04 | DE02 | F1.15 | | | | | | | | |
| | | | C039 | T1.38 | DE03 | T1.12 | | | | | | | | |

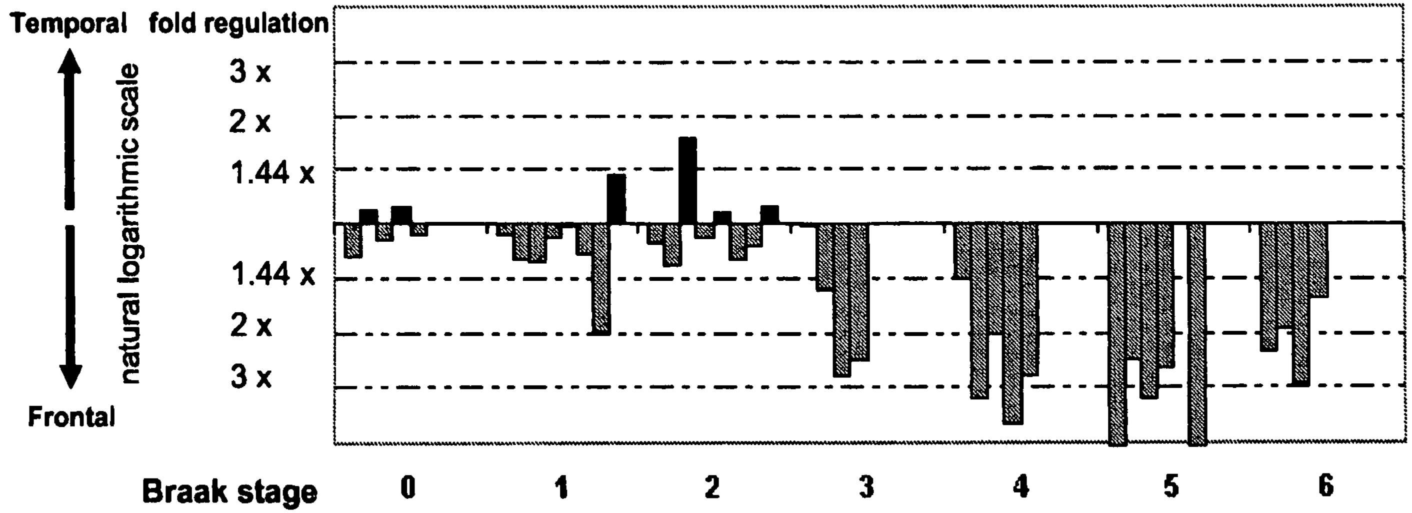

Fig. 13:

| sample | Δ (fold) (hippocampus/ frontal cortex) |
|---|---|
| control C008 | 0.58 |
| control C004 | 0.80 |
| patient P012 | 0.56 |
| patient P016 | 0.16 |
| patient P010 | 0.58 |
| patient P011 | 0.57 |
| patient P014 | 0.70 |
| patient P019 | 0.52 |

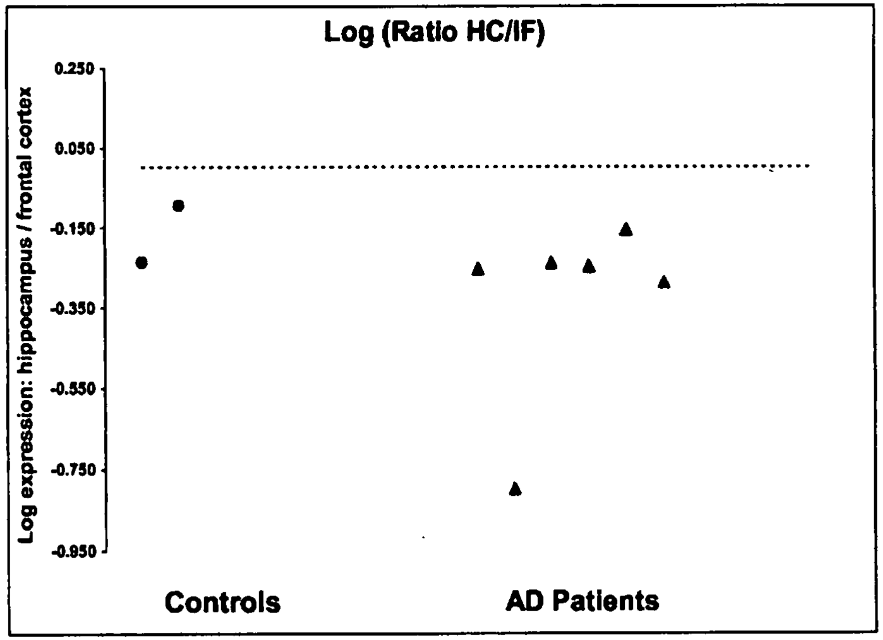

Fig. 14: Images of human brain sections labeled with anti-ATP2B1 antiserum and with DAPI

Control T

Patient T

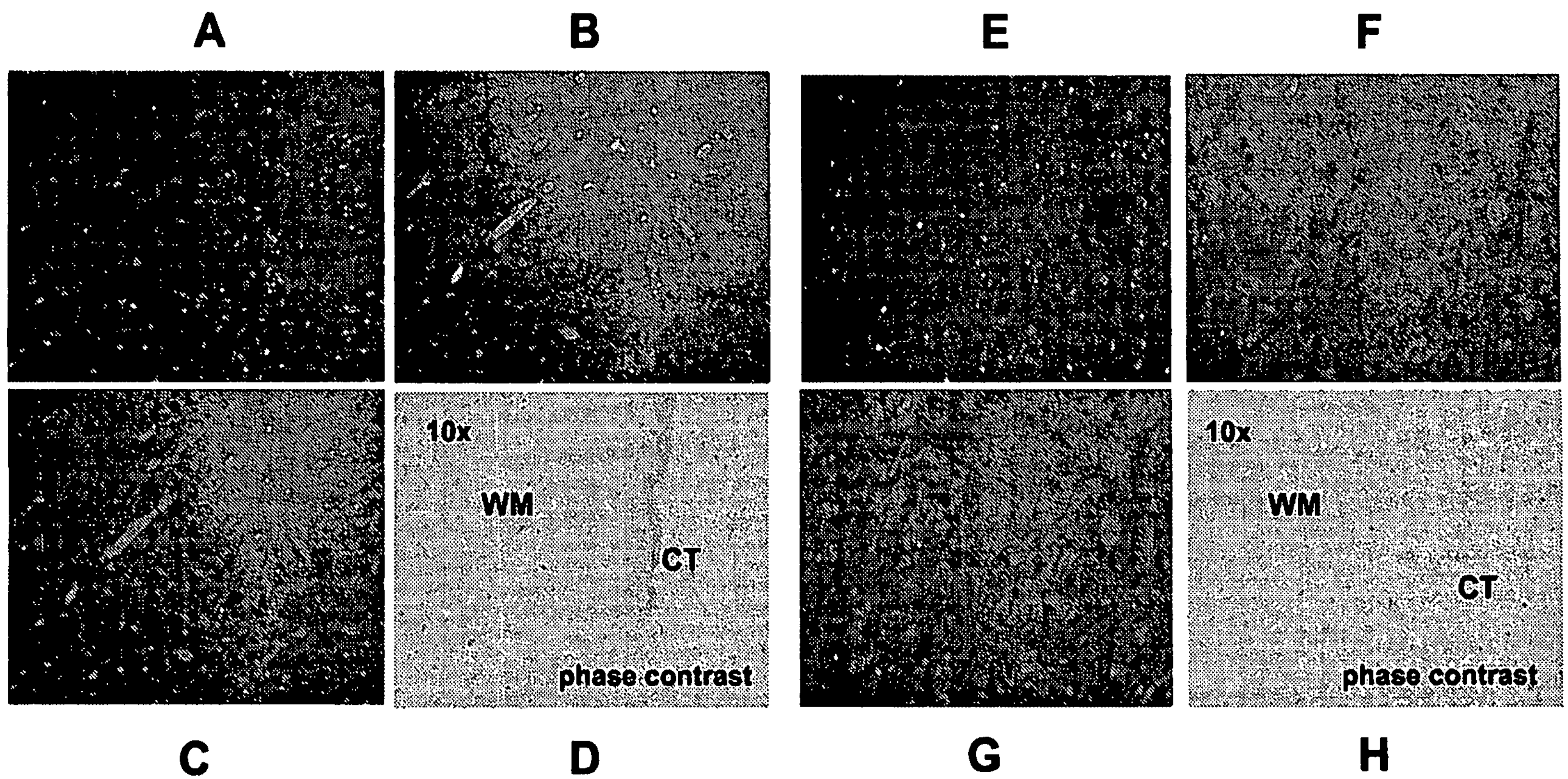

**Fig. 15: Images of human brain sections labeled with anti-ATP2B1 antiserum and with DAPI**

Control F

Patient F

A B E F

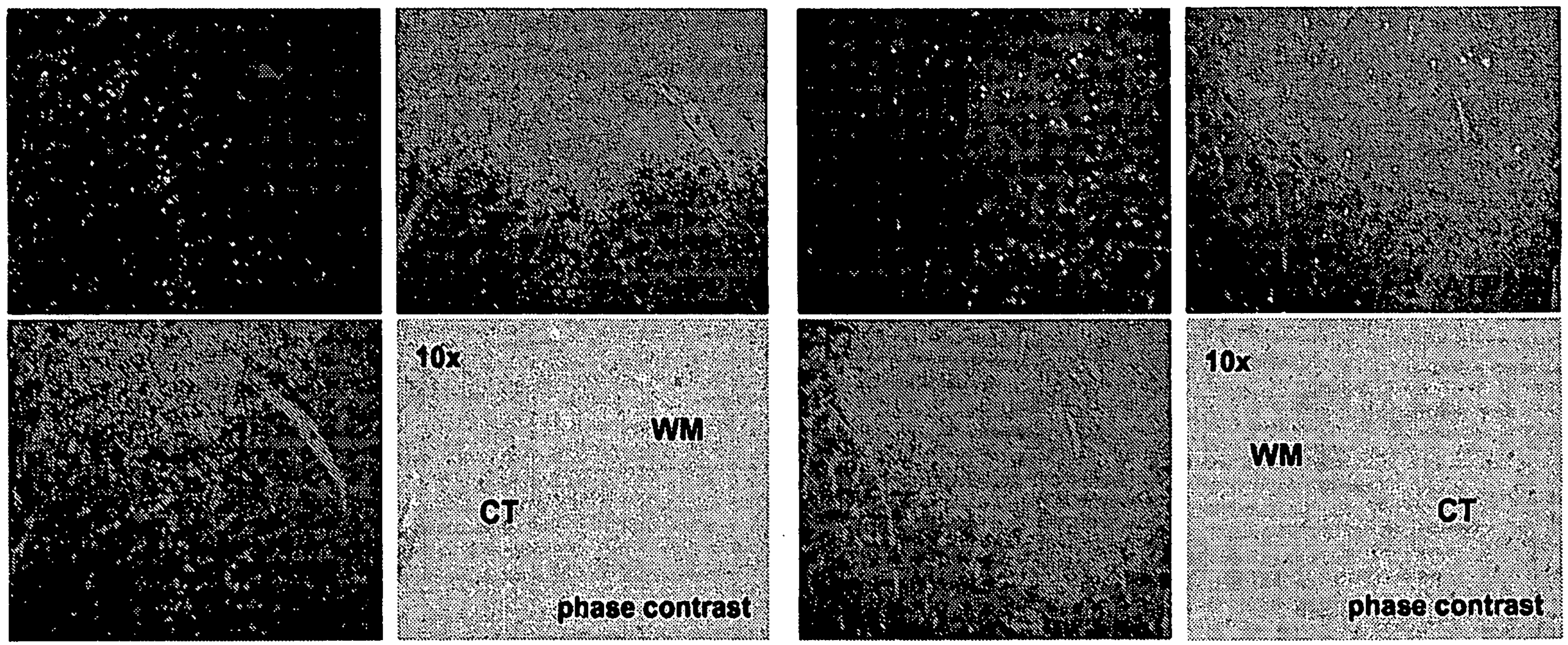


C D G H

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0214543 A
- WO 0052451 A
- WO 0201226 A
- WO 9613744 A
- WO 9816814 A
- WO 9823942 A
- WO 9917086 A
- WO 9934195 A
- WO 0066985 A
- WO 0159436 A
- WO 0159416 A
- US 6150173 A


### Non-patent literature cited in the description


- **VICKERS et al.** *Progress in Neurobiology,* 2000, vol. 60, 139-165
- **WALSH ; SELKOE.** *Neuron,* 2004, vol. 44, 181-193
- **SELKOE ; KOPAN.** *Annu Rev Neurosci,* 2003, vol. 26, 565-597
- **LING et al.** *Int J Biochem Cell Biol,* 2003, vol. 35, 1505-1535
- **BRAAK ; BRAAK.** *J Neural Transm,* 1998, vol. 53, 127-140
- **JOHNSON ; JENKINS.** *J Alzheimers Dis,* 1996, vol. 1, 38-58
- **JOHNSON ; HARTIGAN.** *J Alzheimers Dis,* 1999, vol. 1, 329-351
- **SCHMITT et al.** *Neurology,* 2000, vol. 55, 370-376
- **TERRY et al.** Annals of Neurology. 1981, vol. 10, 184-92
- **STRITTMATTER et al.** *Proc Natl Acad Sci USA,* 1993, vol. 90, 1977-81
- **ROSES.** *Ann NY Acad Sci,* 1998, vol. 855, 738-43
- **STREHLER ; ZACHARIAS.** *Physiological Reviews,* 2001, vol. 81, 21-50
- **HILFIKER et al.** *Journal Biological Chemistry,* 1993, vol. 268, 19717-19725
- **STAUFFER et al.** *Journal Biological Chemistry,* 1993, vol. 268, 25993-26003
- **STAUFFER et al.** *JBC,* 1993, vol. 268, 25993-26003
- **BRANDT et al.** *PNAS,* 1996, vol. 93, 13843-13848
- **BRINI et al.** *Journal Biological Chemistry,* 2001, vol. 278, 24500-24508
- **ALTSCHUL et al.** *J. Mol. Biol.,* 1990, vol. 215, 403-410
- **ALTSCHUL et al.** *Nucleic Acids Res.,* 1997, vol. 25, 3389-3402
- **DEVEREUX et al.** *Nucleic Acids Res.,* 1984, vol. 12, 387
- **PEARSON ; LIPMAN.** *Proc. Natl. Acad. Sci. USA,* 1988, vol. 85, 2444-2448
- **WILBUR ; LIPMAN.** *SIAM J. Appl. Math.,* 1984, vol. 44, 557-567
- **NEEDLEMAN ; WUNSCH.** *J. Mol. Biol.,* 1970, vol. 48, 443-453
- Etiology, Pathogenesis and Therapeutics. **WINBLAD ; WISNIEWSKI.** Alzheimer's Disease and Related Disorders. Wiley & Sons, 1999
- **SCINTO ; DAFFNER.** Early Diagnosis of Alzheimer's Disease. Humana Press, 2000
- **MAYEUX ; CHRISTEN.** Epidemiology of Alzheimer's Disease: From Gene to Prevention. Springer Press, 1999
- **YOUNKIN ; TANZI ; CHRISTEN.** Presenilins and Alzheimer's Disease. Springer Press, 1998
- **BRAAK ; BRAAK.** *Acta Neuropathology,* 1991, vol. 82, 239-259
- **TERRY et al.** Annals of Neurology. 1981, vol. 10, 184-192
- **SAMBROOK ; RUSSELL.** Molecular Cloning: A Laboratory Manual. Cold Spring Harbor Laboratory Press, 2001
- **SCHENA M.** Microarray Biochip Technology. Eaton Publishing, 2000
- **HARLOW ; LANE.** Antibodies: A Laboratory Manual. Cold Spring Harbor Laboratory Press, 1999
- **EDWARDS R.** Immunodiagnostics: A Practical Approach. Oxford University Press, 1999
- **SCHENA M.,.** Microarray Biochip Technology. Eaton Publishing, 2000
- **BEHR.** *Acc Chem Res,* 1993, vol. 26, 274-278
- **MULLIGAN.** *Science,* 1993, vol. 260, 926-931
- **WOLFF.** *Curr Opin Neurobiol,* 1993, vol. 3, 743-748
- **GILLESPIE.** *DN&P,* 1992, vol. 5, 389-395

- **AGRAWAL ; AKHTAR.** *Trends Biotechnol,* 1995, vol. 13, 197-199
- **CROOKE.** *Biotechnology,* 1992, vol. 10, 882-6
- **BARINAGA.** *Science,* 1993, vol. 262, 1512-1514
- **WICKSTROM.** *Trends Biotechnol,* 1992, vol. 10, 281-287
- **HANNON.** *Nature,* 2002, vol. 418, 244-251
- **MC CELL ; PARDEE.** Expression Genetics: Accelerated and High-Throughput Methods. Eaton Publishing, 1999
- **LANZA et al.** *Nature Medicine,* 1999, vol. 9, 975-977
- **PETERSON ; DA.** *Curr. Opin. Pharmacol.,* 2002, vol. 2, 34-42
- **COLMAN A.** *Drug Discovery World,* 2001, vol. 7, 66-71
- **CAPECCHI.** *Science,* 1989, vol. 244, 1288-1292
- **HOGAN et al.** Manipulating the Mouse Embryo: A Laboratory Manual. Cold Spring Harbor Laboratory Press, 1994
- **JACKSO ; ABBOTT.** Mouse Genetics and Transgenics: A Practical Approach. Oxford University Press, 1999
- **SCHWARZ et al.** *Biochemistry,* 1999, vol. 38, 9456-9464
- **KRIVOSHEEV ; USANOV.** *Biochemistry-Moscow,* 1997, vol. 62, 1064-1073
- **HARLOW et al.** Antibodies, A Laboratory Manual. Cold Spring Harbor Laboratory Press, 1988
- **DUBEL ; BREITLING.** Recombinant Antibodies. Wiley-Liss, 1999
- **HARLOW ; LANE.** Using Antibodies: A Laboratory Manual. Cold Spring Harbor Laboratory Press, 1999
- **EDWARDS R.** Immunodiagnostics: A Practical Approach. Oxford University Press, 1999
- **LIANG ; PARDEE.** *Science,* 1995, vol. 267, 1186-7
- **VON DER KAMMER et al.** *Nucleic Acids Research,* 1999, vol. 27, 2211-2218
- **LIANG et al.** *Nucleic Acids Research,* 1994, vol. 22, 5763-5764
- **ZHAO et al.** *Biotechniques,* 1995, vol. 18, 842-850